Europäisches Patentamt

European Patent Office

Office européen des brevets

(11) Publication number: **0 200 090 B1**

(12) **EUROPEAN PATENT SPECIFICATION**

(45) Date of publication of patent specification: **09.12.92**

(51) Int. Cl.5: **A61K 37/02**, A61K 35/48, A61K 35/50, A61K 35/12, C07K 3/02, G01N 33/68

(21) Application number: **86105211.6**

(22) Date of filing: **15.04.86**

The file contains technical information submitted after the application was filed and not included in this specification

(54) **Tissue-derived tumor growth inhibitors, methods of preparation and uses thereof.**

(30) Priority: **19.04.85 US 725003**
**07.04.86 US 847931**

(43) Date of publication of application:
**10.12.86 Bulletin 86/45**

(45) Publication of the grant of the patent:
**09.12.92 Bulletin 92/50**

(84) Designated Contracting States:
**AT BE CH DE FR GB IT LI LU NL**

(56) References cited:
**EP-A- 0 128 849**
**EP-A- 0 136 093**
**FR-A- 2 242 109**

**CHEMICAL ABSTRACTS, vol. 102, no. 12, 25th March 1985, page 313, column 1, abstract no. 100793d, Columbus Ohio, US; GREEN CROSS CORP. "Glycoproteins extraction from human placenta"**

(73) Proprietor: **Oncogene Science, Inc.**
**222 Station Plaza North**
**Mineola New York 11501(US)**

(72) Inventor: **Iwata, Kenneth K.**
**100 S. Grand Street, Apt. 3**
**Westbury New York 11590(US)**
Inventor: **Stephenson, John R.**
**74 Strathmore Lane**
**Rockville New York 11570(US)**
Inventor: **Gold, Leslie I.**
**500 East 83rd Street**
**New York New York 10028(US)**

(74) Representative: **Henkel, Feiler, Hänzel & Partner**
**Möhlstrasse 37**
**W-8000 München 80(DE)**

## Description

This application is a continuation-in-part of U.S. Serial No. 725,003, filed April 19, 1985, the contents of which are hereby incorporated by reference into the present application.

### Background of the Invention

Bichel [Bichel, Nature 231: 449-450 (1971)] reported that removing most of the tumor from mice bearing ascites tumors at a plateau of tumor growth was followed by a marked increase in the growth of the remaining tumor cells. Injection of cell-free ascites, obtained from mice bearing fully developed ascites tumors, into mice with growing ascites tumors resulted in a pronounced inhibition of ascites growth. Bichel, supra, also observed that two surgically joined mice (parabiotic), one mouse with an advanced tumor and the other with an early tumor, resulted in a pronounced inhibition of growth of the early tumor. Based upon these observations, [Bichel, Europ. J. Cancer 6: 291-296 (1970) and Bichel, supra] the existence of a diffusible inhibitory principle which circulated through the peritoneum of the parabiotic mice and was present in the cell-free ascites fluid produced by the fully developed ascites tumors was postulated. The nature of this inhibiting principle was not characterized, but it was speculated that the rate of growth of the ascites tumors was dependent upon the amount of tumor tissue was determined by the amount of inhibitory principle produced.

Substances having tumor growth inhibitory activity have been described. [(Holley, et al., Proc. Natl. Acad. Sci. USA 77:5989 (1980) and Holley, et al., Cell Biol. Int. Reports 7: 525-526 (1983).] These publications report the isolation from African green monkey BSC-1 cells of a growth inhibitory substance which inhibited the growth of BSC-1 cells, human mammary tumor cells and normal human mammary cells. This substance has recently [Tucker, et al., Science 226: 705-707 (1984): Roberts, et al. Proc. Nat. Acad. Sci. 82 (Jan): 119-123 (1985)] been shown to be identical, or highly related, to a 25,000 dalton two chain human platlet-de-rived polypeptide designated B-TGF (Assoian, et al., J. Biol. Chem. 258: 7155-7160 (1983 EP-A-0128849]. Independently, McMahon, et al. [Proc. Natl. Acad. Sci. USA 79, 456-460 (1982)] have purified from rat liver a 26,000 dalton substance which inhibits the proliferation of nonmalignant rat liver cells, but does not inhibit the proliteration of malignant rat liver cells. Other growth inhibitory substances have been identified in cultured chick spinal cord cells [Kagen, et al., Experimental Neurology 58: 347-360 (1970); Harrington, et al., Proc. Natl. Acad. Sci. USA 77: 423-427 (1980) and Steck, et al., J. Cell Biol. 83: 562-575 (1979).]

Iwata, et al., [J. Cellular Biochem. Suppl. 5: 401 (1982)] previously described a microtiter plate system for assaying growth stimulation and growth inhibition activity. Todaro, et al. [Todaro, et al., in Tumor Cell Heterogeneity; Origins and Implications, Bristol-Myers Cancer Symposia, Volume 4, Owens, A.H., Coffey, D.S., and Baylin, S.B., Eds. (Academic Press, 1982), pp. 205-224] and Iwata, et al. [Fed. Proc. Fed. AM. Soc. Exp. Biol. 42: 1833 (1983)] previously reported the isolation of tumor inhibitory activity from tissue culture fluids of human tumor cells propagated in culture. The observations described in these reports were preliminary and little detail was provided.

On April 20, 1984, a patent application was filed with the United States Patent and Trademark Office under U.S. Serial No. 602,520, entitled "Substantially Purified Tumor Growth Inhibitory Factor (TIF)" on which one of us, Kenenth K. Iwata, is named as coinventor. This application concerns the preliminary identification of a not well-defined substance or substances present in, and derived from, human tumor cells propagated in culture. This substance or substances resembles the tumor inhibitory activity previously reported. [Todaro, et al., in Tumor Cell Heterogeneity; Origins and Implications, Bristol-Myers Cancer Symposia, Volume 4, Owens, A.H., D.S., and Baylin, S.B., Eds. (Academic Press, 1982), pp. 205-224; Iwata, et al., Fed. Proc. Fed. Am. Soc. Exp. Biol. 42: 1833 (1983).]

Todaro [Todaro, G.J. in Epigenetic Regulation of Cancer, Terry Fox Cancer Research Conference (University of British Columbia; Vancouver, B.C., Canada) Abs. 13 (1984)] recently reported two factors with tumor cell growth inhibitory properties which were reportedly sequenced and shown to consist of 70 and 90 amino acid residues, respectively. Neither the source, i.e. cell, tissue type of species, of these factors or the method of purification of the factors were disclosed.

FR-A 74 29570 describes a process for the preparation of a tissue derived growth inhibitor as therapeutic agent against leukemia. Said growth inhibitor is obtained by extraction of the placenta and is called the D-factor. The D-factor is thermally very stable and can be sterilized at 110°C during 15 minutes or at 130°C during 10 minutes under water pressure.

### Summary of the Invention

The present invention provides a composition of matter designated tissue-derived growth inhibitor-1 (TGI-1). TGI-1 comprises at least one polypeptide having the property of inhibiting the growth of human tumor cells and of an established mink lung cell line (CCL64) but not the growth of normal human foreskin fibroblasts and having an apparent molecular weight in the range from 5,000-16,000 daltons.

TGI-1 is derivable from an acidified ethanol extract from human tissue which comprises a plurality of acidic polypeptides, each of which has a molecular weight of less than 20,000 daltons and each of which has the property of inhibiting the growth of human tumor cells and of an established mink lung cell line (CCL 64) while stimulating the growth of normal human foreskin fibroblasts, the inhibitory activity against human tumor cell growth not being destroyed upon increasing the temperature of the acidified, ethanol extract to 100°C for 3 minutes or upon adding acetic acid until the acidified, ethanol extract is up to 1.0 molar in acetic acid and the inhibitory activity being enhanced when the acidified, ethanol extract is prepared at 4°C rather than at 23°C.

Also, TGI-1 is recoverable as a defined activity on high performance liquid chromatography of an acidified, ethanol extract with a linear gradient of acetonitrile containing 0.05% trifluoroacetic acid at 26-34% acetonitrile. Further, TGI-1 is recoverable as a defined activity on high performance liquid chromatography of the acidified, ethanol extract with a linear gradient of 2-propanol containing 0.05% trifluoroacetic acid at 17-23% 2-propanol.

The present invention further provides a composition of matter designated tissue-derived growth inhibitor-2 (TGI-2).

TGI-2 comprises at least one polypeptide having the property of inhibiting the growth of human tumor cells and of an established mink lung cell line (CCL 64) but not the growth of normal human foreskin fibroblasts and having an apparent molecular weight in the range from 5,000-16,000 daltons.

Additionally, TGI-2 is recoverable as a defined activity on high performance liquid chromatography of an acidified ethanol extract derived from human tissue. The extract comprises a plurality of acidic polypeptides, each of which has an apparent molecular weight less than 20,000 daltons and each of which has the property of inhibiting the growth of human tumor cells and of an established mink lung cell line (CCL 64) while stimulating the growth of normal human foreskin fibroblasts, the inhibitory activity against human tumor cell growth not being destroyed upon increasing the temperature of the acidified, ethanol extract to 100°C for 3 minutes or upon adding acetic acid until the acidified, ethanol extract is up to 1.0 molar in acetic acid and the inhibitory activity being enhanced when the acidified, ethanol extract is prepared at 4°C rather than at 23°C.

Moreover, TGI-2 is recoverable as a defined activity on high performance liquid chromatography with a linear gradient of acetonitrile containing 0.05% trifluoroacetic acid at 35-39% acetonitrile and being recoverable as a defined activity on high performance liquid chromatography of acidified, ethanol extract with a linear gradient of 2 propanol containing 0.05% trifluoroacetic acid at 23-27% 2-propanol.

Additionally, this invention provides a method for preparing a composition of matter designated tissue-derived growth inhibitor-1 (TGI-1) which comprises first preparing an acidified, ethanol extract and then recovering TGI-1 from the acidified, ethanol extract as a defined activity by high performance liquid chromatography of the acidified, ethanol extract with first i) a linear gradient of acetonitrile containing 0.05% trifluoroacetic acid at 26-34% acetonitrile and then ii) a linear gradient of 2-propanol containing 0.05% trifluoroacetic acid at 17-23% 2-propanol, wherein said acidified, ethanol extract is prepared from human tissue, the acidified, ethanol extract comprising a plurality of polypeptides, each of which has a molecular weight less than 20,000 daltons and each of which has the property of inhibiting the growth of human tumor cells and of an established mink lung cell line (CCL64) while stimulating the growth of normal human foreskin fibroblasts.

The method of preparing an acidified, ethanol extract comprises, under suitable conditions, the following steps: a) treating the tissue to produce lysed cells and removing the solubilized proteins derived from the cells; b) recovering the solubilized proteins; c) separately recovering from the solubilized proteins polypeptides having an apparent molecular weight less than 20,000 daltons; d) assaying the separately recovered polypeptides to identify those which inhibit the growth of human tumor cells and inhibit the growth of an established mink lung cell line (CCL64) while enhancing the growth of normal human foreskin fibroblasts; and e) recovering an acidified, ethanol extract containing the polypeptides so identified.

Also, this invention provides a method for preparing a composition of matter designated tissue-derived growth inhibitor-2 (TGI-2) which comprises first preparing an acidified, ethanol extract and then recovering TGI-1 from the acidified, ethanol extract as a defined activity by high performance liquid chromatography of the acidified, ethanol extract with first i) a linear gradient of acetonitrile containing 0.05% trifluoroacetic acid at 35-37% acetonitrile and then ii) a linear gradient of 2-propanol containing 0.05% trifluoroacetic acid at 23-27% 2-propanol, wherein said acidified, ethanol extract is prepared from human tissue, the acidified, ethanol extract comprising a plurality of polypeptides, each of which has a molecular weight less than 20,000 daltons and each of which has the property of inhibiting the growth of human tumor cells and of an established mink lung cell line (CCL64) while stimulating the growth of normal human foreskin fibroblasts.

The method of preparing an acidified, ethanol extract comprises, under suitable conditions, the following steps: a) treating the tissue to produce lysed cells and removing the solubilized proteins derived from the cells; b) recovering the solubilized proteins; c) separately recovering from the solubilized proteins polypeptides having an apparent molecular weight less than 20,000 daltons; d) assaying the separately recovered polypeptides to identify those which inhibit the growth of human tumor cells and inhibit the growth of an established mink lung cell line (CCL64) while enhancing the growth of normal human foreskin fibroblasts; and e) recovering an acidified, ethanol extract containing the polypeptides so identified.

Further, this invention provides a method for detecting the presence of a tumor which comprises quantitatively determining the amount of TGI-1 present in a sample from a subject and comparing the amount so determined with the amount present in a sample from a normal subject, the presence of a significantly different amount indicating the presence of a tumor.

Additionally, this invention provides a method for detecting the presence of a tumor which comprises quantitatively determining the amount of TGI-2 present in a sample from a subject and comparing the amount so determined with the amount present in a sample from a normal subject, the presence of a significantly different amount indicating the presence of a tumor.

The present invention also provides a method for detecting the presence of a tumor which comprises quantitatively determining the amount of TGI-1 and of transforming growth factor alpha (TGF-alpha) present in a sample from a subject, determining the ratio of the amount of TGI-1 present in the sample to the amount of TGF-alpha, determining the comparable ratio for a sample from a normal subject and comparing the ratio for the normal subject, a significantly different amount indicating the presence of a tumor.

This invention further provides a method for detecting the presence of a tumor which comprises quantitatively determining the amount of TGI-2 and of transforming growth factor alpha (TGF-alpha) present in a sample from a subject, determining the ratio of the amount of TGI-2 present in the sample to the amount of TGF-alpha, determining the comparable ratio for a sample from a normal subject and comparing the ratio for the normal subject, a significantly different amount indicating the presence of a tumor.

Brief Description of the Figures

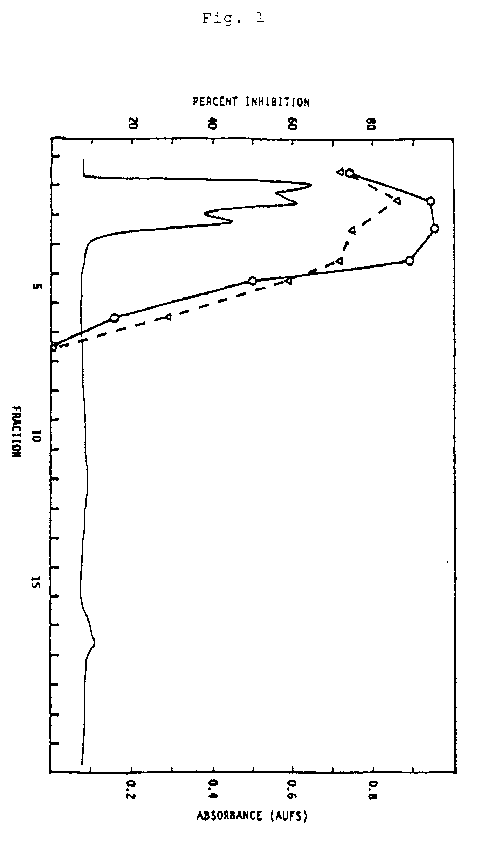

Fig. 1. Gel filtration chromatography at 23°C

Elution pattern of gel filtration chromatography at 23°C of crude acidified, ethanol extract from human umbilical cords. Two grams of acidified, ethanol extract in 150 ml of 1.0 M acetic acid was applied to a 14 x 100 cm column (Amicon; #86012) containing Bio-Gel P10 and eluted at a flow rate of 7 ml/min. One liter fractions were collected on a SuperRac (LKB 2211) equipped with a type C collection rack (LKB). One ml aliquots of each fraction (1 liter/fraction) were transferred to 12 x 75 mm sterile snap top tubes (Falcon 2058). TGI activity was determined as described in Materials and Methods. Inhibition of A549 human lung carcinoma cells is shown by open triangles and mink lung (CCL64) cells by open circles. Absorbance at 280 nm

( ——————— )

was detected by a Uvicord S (LKB 2138) with a full scale absorbance range of 1.0 AUFS and a single channel chart recorder (LKB 2210) with a chart speed of 1 mm/min.

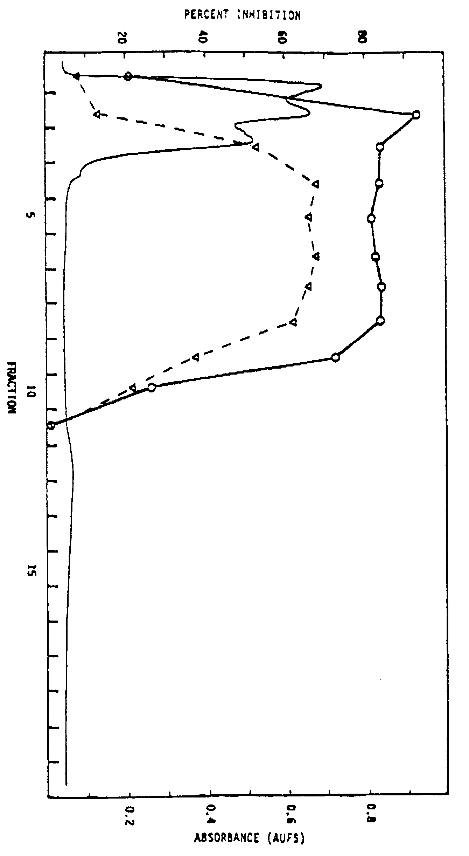

Fig. 2. Gel filtration Chro matography at 4°C

Elution pattern of gel filtration chromatography at 4°C of crude acidified, ethanol extract from human umbilical cords. Two grams of acidified, ethanol extract in 150 ml of 1.0 M acetic acid was applied to a 14 x 100 cm column (Amicon; #86012) containing Bio-Gel P10 and eluted at a flow rate of 7 ml/min. One liter fractions were collected on a SuperRac (LKB 2211) equipped with a type C collection rack (LKB). One ml aliquots of each fraction (1 liter/fraction) were transferred to 12 x 75 mm sterile snap top tubes (Falcon 1058). TGI activity was determined as described in Materials and Methods. Inhibition of A549 human lung carcinoma cells is shown by open triangles and mink lung (CCL64) cells by open circles. Absorbance of 280 nm

( ————— )

was detected by a Uvicord S (LKB 2138) with a full scale absorbance range of 1.0 AUFS and a single channel chart recorder (LKB 2210) with a chart speed of 1 mm/min.

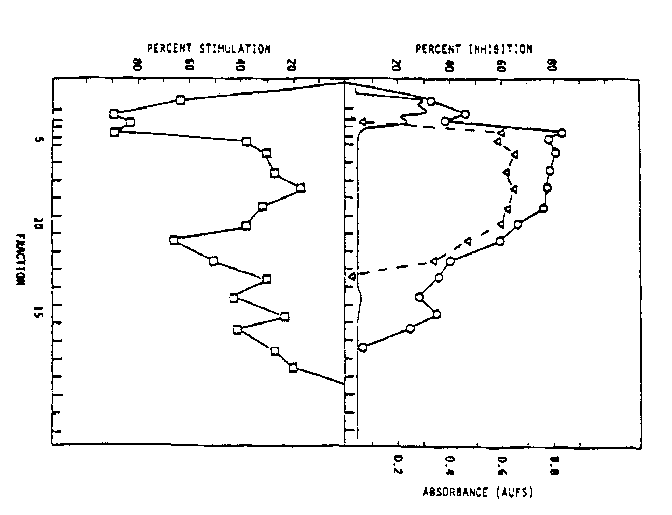

Fig. 3. Cell growth inhibition and normal human cell stimulation by fractions from gel filtration chromatography at 4°C

Elution pattern of gel filtration chromatography at 4°C of crude acidified, ethanol extract in 150 ml of 1.0 M acetic acid was applied to a 14 x 100 cm column (Amicon; #86012) containing Bio-Gel P10 and eluted at a flow rate of 7 ml/min. One liter fractions were collected on a SuperRac (LKB 2211) equipped with a type C collection rack (LKB) . One ml aliquots of each fraction (1 liter/fraction) were transferred to 12 x 75 mm sterile snap top tubes (Falcon 2058). TGI activity was determined as described in Materials and Methods. Inhibition of A549 human lung carcinoma cells is shown by open triangles and mink lung (CCL64) cells by open circles. Stimulation of normal human fibroblasts is shown by open squares. Absorbance of 280 nm

( ——— )

was detected by Uvicord S (LKB 2138) with a full scale absorbance range of 1.0 AUFS and a single channel chart recorder (LKB 2210) with a chart speed of 1 mm/min.

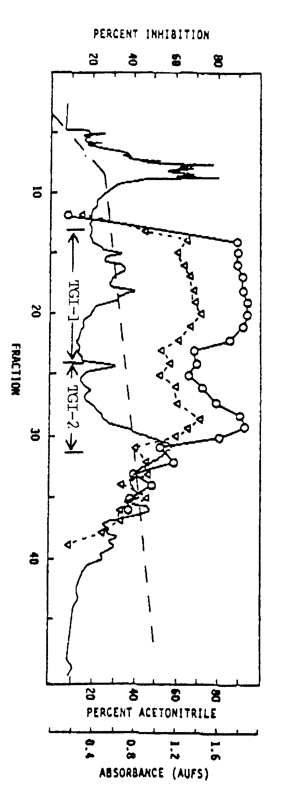

Fig. 4. Reverse phase high performance liquid chromatography (HPLC) of an active fraction from gel filtration chromatography

Fraction 4 derived from gel filtration chromatography on Bio-Gel P10 of human umbilical cord acidified, ethanol extract (65.8 mg protein) was lyophilized and resuspended in 10 ml of 0.05% trifluoroacetic acid (TFA). Fraction 4 was the first fraction following the major peaks of absorbance at 280 nm. (Figure 2) The sample was centrifuged on a Beckman table top centrifuge (Beckman TJ-6) at 3000 rpm for 20 minutes to remove insoluble material. Three separate injections of the supernatant were made through a Water's U6K injector equipped with a 2 ml sample loop. The sample was then loaded onto a uBONDAPAK $C_{18}$ column (0.78 x 30 cm) (Waters #84176). The flow rate was 2 ml/min. and the effluent monitored at 206 nm

( ————— )

with a Waters u.v. detector (Waters Model 481) at a sensitivity of 2.0 AUFS. Elution was achieved with a linear 30-min gradient from 0.25% of increasing concentrations of acetonitrile containing 0.05% TFA, followed by a linear 240-min gradient of 25-45% acetonitrile containing 0.05% TFA, followed by a linear 30-min gradient of 45-100% acetonitrile containing 0.05% TFA. A SuperRac (LKB 2211) was used to collect 12 ml fractions. One ml aliquots of each fraction were transferred to 12 x 75 mm polystyrene tubes (Falcon 2058) containing 50 ul of 1.0 M acetic acid and 50 ug of bovine serum albumin (Sigma B) and assayed for TGI activity as described in Materials and Methods. Inhibition of A549 human lung carcinoma cells is shown by open triangles and of mink lung (CCL 64) by open circles. The solvent gradient is shown by large dashes

( —— —— —— ) .

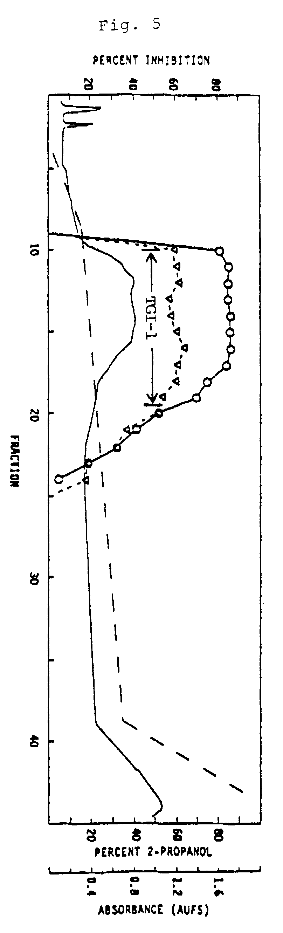

Fig. 5. HPLC rechromatography of pooled TGI activity from HPLC (TGI-1)

Pooled fractions of TGI activity (1.5 mg) eluting between 28-34% acetonitrile (fractions 13-22) by HPLC chromatography (Figure 4) was lyophilized and resuspended in 2 ml of 0.05% TFA. The sample was centrifuged on a Beckman table top centrifuge (Beckman TJ-6) at 3000 rpm for 20 minutes to remove insoluble material. Two separate injections of the supernatant were made through a Water's U6K injector equipped with a 2 ml sample loop. The sample was loaded onto a uBONDAPAK $C_{18}$ column (0.39 x 30 cm) (Waters #27324). The flow rate was 1 ml/min. and the effluent monitored at 206 nm

( ——————— )

with a Waters u.v. detector (Waters Model 481) at a sensitivity of 2.0 AUFS. Elution was achieved with a linear 20-min gradient from 0-15% of increasing concentrations of 2-propanol containing 0.05% TFA, followed by a linear 120-min gradient of 15-35% 2-propanol containing 0.05% TFA. A SuperRac (LKB 2211) was used to collect 4 ml fractions. One ml aliquots of each fraction were transferred to 12 x 75 mm polystyrene tubes (Falcon 2058) containing 50 ul of 1.0 M acetic acid and 50 ug of bovine serum albumin (Sigma A-6003) and assayed for TGI activity as described in Materials and Methods. Inhibition of A549 human lung carcinoma cells is shown by open triangles and of mink lung (CCL64) cells by open circles. The solvent gradient is shown by large dashes

( —— —— —— ) .

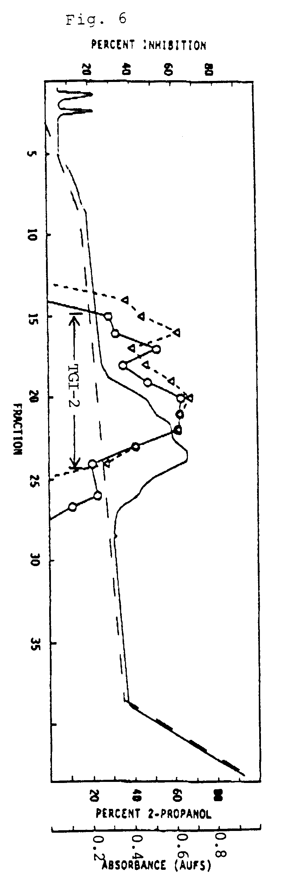

Fig. 6. Reverse phase HPLC rechromatography of pooled activity from HPLC (TGI-2)

Pooled fractions of TGI activity (0.8 mg) eluting between 35-39% acetonitrile (fractions 25-31) by HPLC chromatography (Figure 4) was lyophilized and resuspended in 2 ml of 0.05% TFA. The sample was centrifuged on a Beckman tabletop centrifuge (Beckman TJ-6) at 3000 rpm for 30 min to remove insoluble material. Two separate injections of the supernatant were made through a Water's U6K injector equipped with a 2 ml sample loop. The sample was loaded onto a uBONDAPAK $C_{18}$ column (0.39 x 30 cm) (Waters 27324). The flow rate was 1 ml/min and the effluent monitored at 206 nm

( ——————— )

with a Waters u.v. detector (Waters Model 481) at a sensitivity of 1.0 AUFS. Elution was achieved with a linear 20-min gradient from 0.15% of increasing concentrations of 2-propanol containing 0.05% TFA, followed by a linear 120-min gradient of 15-35% 2-propanol containing 0.05% TFA. A SuperRac (LKB 2211) was used to collect 4 ml fractions. One ml aliquots of each fraction were transferred to 12 x 75 mm polystyrene tubes (Falcon 2058) containing 50 ul of 1.0 M acetic acid and 50 ug of bovine serum albumin (Sigma A-6003) and assayed for TGI activity as described in Materials and Methods. Inhibition of A549 human lung carcinoma cells is shown by open triangles and of mink lung (CCL64) cells by open circles. The solvent gradient is shown by large dashes

( —— —— —— ) .

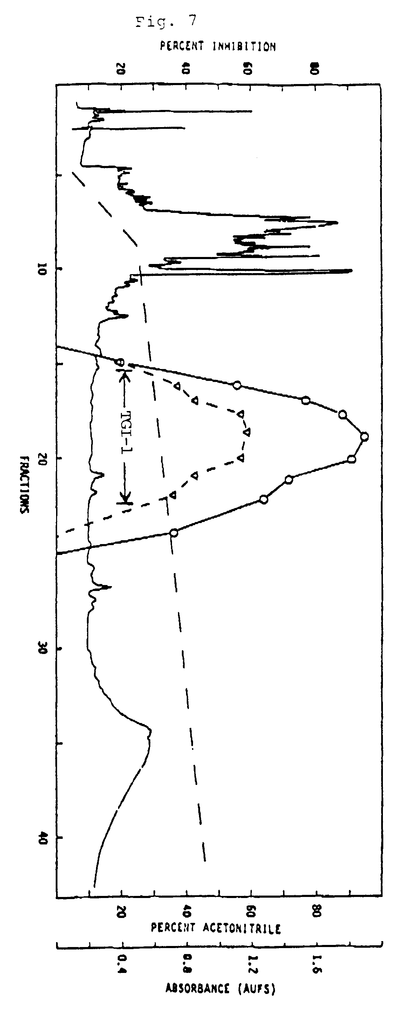

Fig. 7. Reverse phase HPLC of an active fraction from gel filtration chromatography

Fraction 5 derived from gel filtration chromatography on Bio-Gel P10 of human umbilical cord acidified, ethanol extract was lyophilized and resuspended in 4 ml of 0.05% trifluoroacetic acid. (TFA). Fraction #5 was the second fraction following the major peaks of absorbance at 280 nm. (Figure 2) The sample was centrifuged on a Beckman tabletop centrifuge (Becman TJ-6) at 3000 rpm for 20 min to remove insoluble material. Two separate injections of the supernatant were made through a Water's U6K injector equipped with a 2 ml sample loop. The sample (1.3 ml total) was then loaded onto a uBONDAPAK $C_{18}$ column (0.78 x 30 cm) (Waters #84176). The flow rate was 2 ml/min and the effluent monitored at 206 nm

( ——— )

with a Waters u.v. detector (Waters Model 481) with a sensitivity of 2.0 AUFS. Elution was achieved with a linear 30-min gradient from 0.25% of increasing concentrations of acetonitrile containing 0.05% TFA, followed by a linear 240-min gradient of 25-45% acetonitrile containing 0.05% TFA, followed by linear 30-min gradient of 45-100% acetonitrile containing 0.05% TFA. A SuperRac (LKB 2211) was used to collect 12 ml fractions. One ml aliquots of each fraction were transferred to 12 x 87 mm polystyrene tubes (Falcon 2058) containing 50 ul of 1.0 M acetic acid and 50 ug of bovine serum albumin (Sigma A6003) and assayed for TGI activity as described in Materials and Methods. Inhibition of A549 human lung carcinoma cells is shown by open triangles and of mink lung (CCL 64) cells by open circles. The solvent gradient is shown by large dashes

$$(— \; — \; —).$$

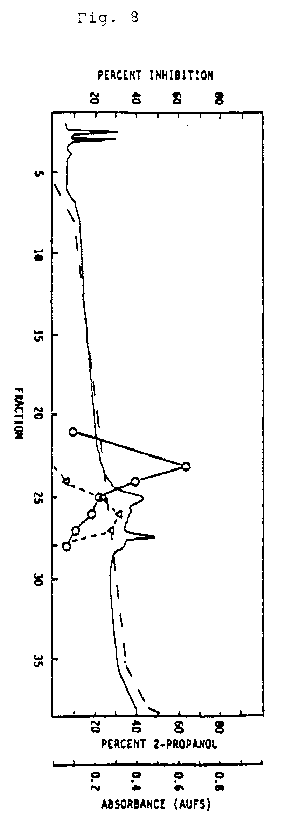

Fig. 8. Reverse phase HPLC rechromatography of pooled TGI activity from HPLC (TGI-1)

Pooled fractions of TGI activity (1.1 mg) eluting between 29-34% acetonitrile (Figure 7; fractions 14-25) were lyophilized and resuspended in 2 ml of 0.05% TFA. The samples were centrifuged on a Beckman tabletop centrifuge (Beckman TJ-6) at 3000 rpm for 20 minutes to remove insoluble material. Two separate injections of the supernatant (1.6 ml) were made through a Water's U6K injector equipped with a 2 ml sample loop. The sample was then loaded onto a uBONDAPAK $C_{18}$ column (0.78 x 30 cm) (Waters 84174). The flow rate was 1 ml/min and the effluent monitored at 206 nm

$$(————)$$

with a Waters u.v. detector (Waters Model 481) with a sensitivity of 1.0 AUFS. Elution was achieved with a linear gradient for 20 min from 0-10% of increasing concentrations of 2-propanol containing 0.05% TFA, followed by a linear 220-min gradient of 10-35% 2-propanol containing 0.05% TFA, followed by a linear 20-min gradient of 35-45% 2-propanol containing 0.05% TFA, followed by a linear 20-min gradient of 45-100% 2-propanol containing 0.05% TFA. A SuperRac (LKB 2211) was used to collect 8 ml fractions. One ml aliquots of each fraction were transferred to 12 x 75 mm polystyrene tubes (Falcon 2058) containing 50 ul of 1.0 M acetic acid and 50 ug of bovine serum albumin (Sigma 6003) and assayed for TGI activity as previously described. Inhibition of A549 human lung carcinoma cells is shown by open triangles and of mink lung (CCL64) cells by open circles. The solvent gradient is shown by large dashes

$$(— \; — \; —).$$

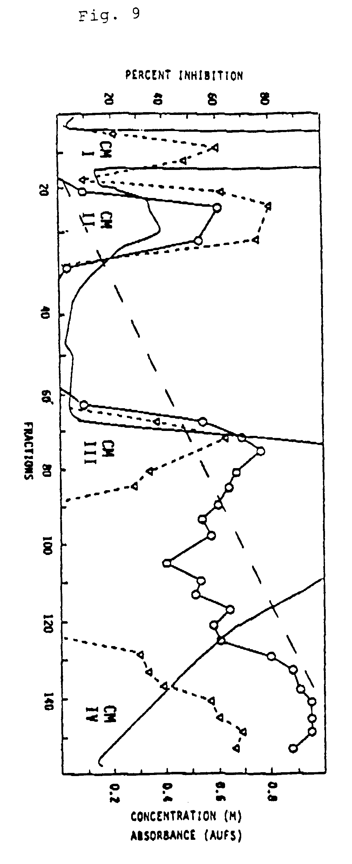

Fig. 9. Cation exchange chromatography of human umbilical cord extracts

CM-TRISACRYL was resuspended in an equal volume of 0.1 M ammonium acetate, pH 4.0, containing 1.0 M NaCl. The resin was allowed to equilibrate for 3 hours and degassed at 4°C. Twenty ml of resin was packed into a 1.6 x 40 cm column (Pharmacia; #19-0362-01) and washed with 2 column volumes of 1.0 M ammonimum acetate pH 4.0, followed by 0.01 M ammonium acetate. The column was washed until the effluent matched the conductivity and the pH of the equilibrating buffer (0.01 M ammonium acetate pH 4.0). One gram of human umbilical cord acidified, ethanol extract was resuspended in 50 ml of 1.0 M acetic acid and dialyzed against the column equilibration buffer at 4°C until the pH and the conductivity matched that of the equilibration buffer. The dialyzed acidified, ethanol extract was applied to the column at a flow rate of 1 ml/min at 4°C and the column was washed with the equilibrating buffer until the absorbance

$$(————),$$

A280, as monitored by a Uvicord S (LKB 2138) with a sensitivity of 1.0 AUFS, was at its lowest point. This was followed by 200 ml of an ascending molarity linear gradient from 0.01 to 1.0 M ammonium acetate, pH 4.0, which was applied using a gradient mixer (Pharmacia GM-1, #19-0495-01). At the end of the gradient, an additional 30 ml of 1.0 M ammonium acetate, pH 4.0, were passed through the column. Two ml fractions were collected in 12 x 100 mm polystyrene tubes (Columbia Diagnostics B-2564) in a SuperRac fraction collector (LKB 2211). One ml aliquots from each fraction were transferred to 12 x 75 mm tubes (Falcon 2058) containing 50 ul 1.0 M acetic acid and 50 ug bovine serum albumin (Sigma A6003), lyophilized, and assayed for TGI activity as described in Materials and Methods. Inhibition of A549 human lung carcinoma cells is shown by open triangles and of mink lung (CCL64) cells by open circles. The salt gradient is shown by large dashes

$$( \text{——} \quad \text{——} \quad \text{——}) \; .$$

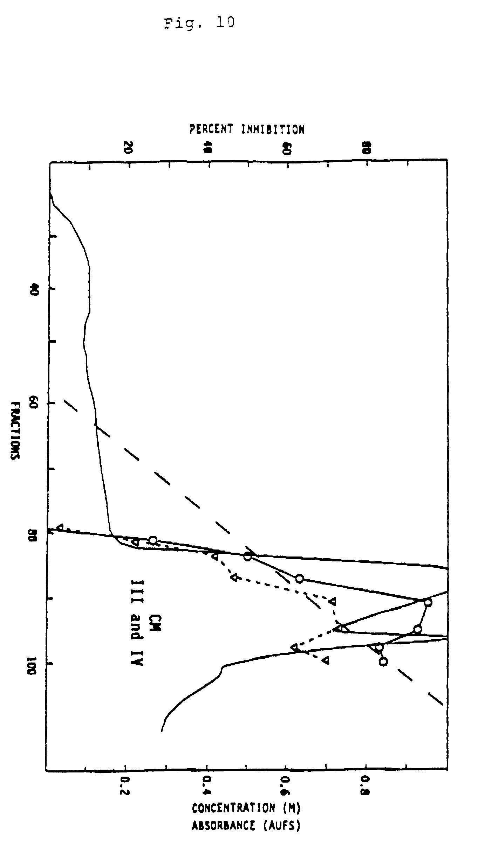

Fig. 10 Rechromatography of a pooled fraction from cation exchange chromatography

CM-TRISACRYL was prepared as described in Figure 9. The material from fractions containing CM III and CM IV were pooled, lyophilized, resuspended in 50 ml of 0.1 M acetic acid and dialyzed against the column equilibration buffer at 4°C until the pH and the conductivity matched that of the equilibration buffer. The sample was applied to the column at a flow rate of 1 ml/min at 4°C and the column was washed with 120 ml of the equilibrating buffer. Absorbance

$$(\text{————————})$$

(A280) was monitored by a Uvicord S (LKB 2138) with a sensitivity of 1.0 AUFS. One hundred ml of an ascending molarity linear gradient from 0.01 to 1.0 M ammonium acetate, pH 4.0, was applied using a gradient mixer (Pharmacia; GM-1, #19-0495-01). At the end of the gradient, an additional 30 ml of 1.0 M amonium acetate, pH 4.0, was passed through the column. Two ml fractions were collected in 12 x 100 mm polystyrene tubes (Columbia Diagnostics B2564) in a SuperRac fraction collector (LKB 2211). One ml aliquots from each fraciton were transferred to 12 x 75 mm tubes (Falcon 2058) containing 50 ul 1.0 M acetic acid and 50 ug bovine serum albumin (Sigma A6003), lyophilized, and assayed for TGI activity as described in Materials and Methods. Inhibition of A549 human lung carcinoma cells is shown by open triangles and mink lung (CCL64) cells by open circles. The salt gradient is shown by large dashes

$$( \text{——} \quad \text{——} \quad \text{——}) \; .$$

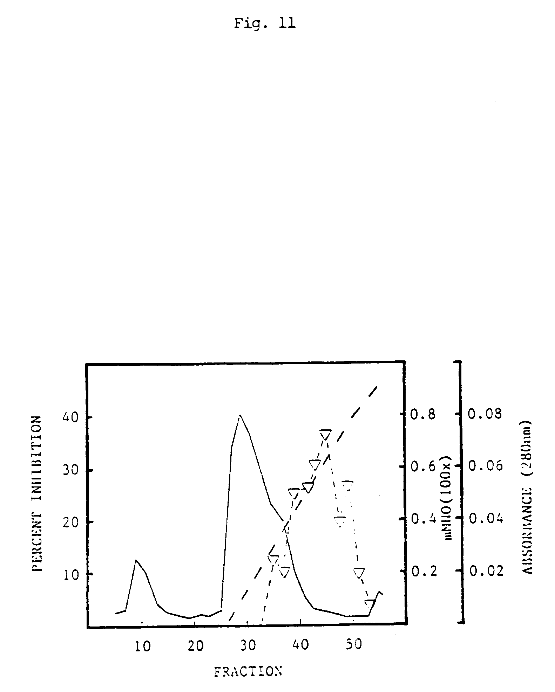

Fig. 11. Fractionation of TGI by Cation Exchange Chromatography at 4°C

1.65 mg of protein extract prepared as described in the Second Series of Experiments was dialysed extensively against 20mM ammonium acetate (pH 4.5) and applied to a 5 ml (1 x 6.3 cm) column of CM-TRISACRYL previously equilibrated in 20 mM ammonium acetate (pH 4.5) and 1.65 ml fractions (12 x 100 mm polystyrene tubes) were collected. Following sample application, the column was washed with 20 mM ammonium acetate, pH 4.5, until the absorbance at 280 nm (0-0) returned to baseline values (less than 0.003) as determined with a Bausch and Lomb 1001 spectrophotometer using a 1 cm light path quartz cuvet. A linear salt gradient (0-1.0 M NaCl in 20 mM ammonium acetate, pH 4.5) was applied and the absorbance at 280 nm of the 1.65 ml fractions was determined as described above. 10 ul aliquots of the indicated fractions were transferred to 12 x 75 mm tubes containing 50 ul 1.0 M acetic acid and 50 ug bovine serum albumin (Sigma A6003), lyophilized, and assayed for inhibitory activity

$$( \triangledown\text{-- } \text{-- --}\triangledown )$$

against A549 human lung carcinoma cells as described under Materials and Methods. The NaCl gradient

$$( \text{——} \quad \text{——} \quad \text{——} \quad \text{——})$$

was determined by measuring the conductivity (YSI Model 32 Conductance Meter) of suitable samples diluted 100-fold in $H_2O$.

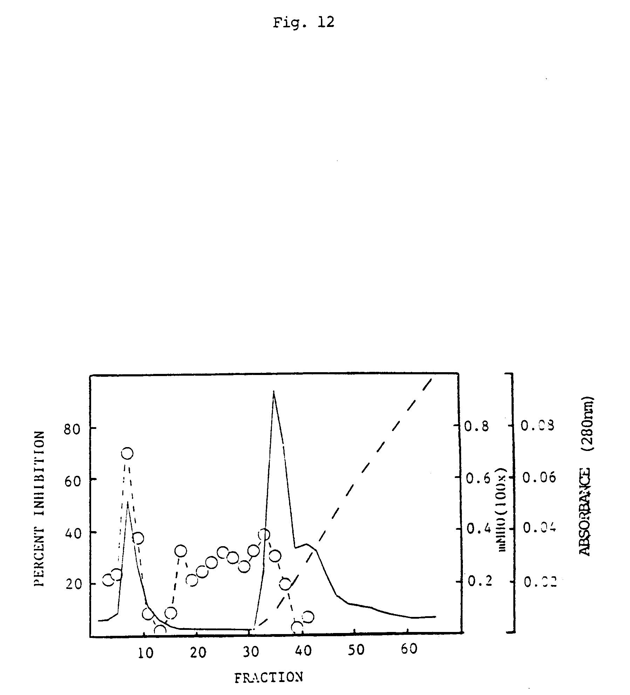

Fig. 12. Fractionation of TGI by Anion Exchange Chromatography at 4°C

1.65 mg of protein extract prepared as described in the Second Series of Experiments was dialyzed extensively against 20 mM Tris-HCl (pH 8.0) and clarified by centrifugation at 3,000 x g for 15 minutes. DEAE-TRISACRYL was prepared by suspending the resin first in 20 mM Tris, HCl (pH 8.0) containing 1.0 M NaCl for 3 hours and secondly in 0.5 M Tris, HCl (pH 8.0) for 1 hour. The sedimented resin was washed on a buchner funnel with 1000 ml $H_2O$ and finally resuspended in 20 mM Tris, HCl (pH 8.0), degassed and poured into a 5 ml column (1 x 6.3 cm) and the resin equilibrated with 20 mM Tris, HCl (pH 8.0) . The clarified sample was applied to the column and absorbance at 280 nm

( ——————— ) ,

inhibitory activity against mink lung cells (0-0), and the NaCl gradient

(— — —)

was determined as described in Fig. 11 and under Materials and Methods. The linear NaCl gradient in 20 mm Tris, HCl (pH 8.0) ranged from 0 to 1.0 M NaCl.

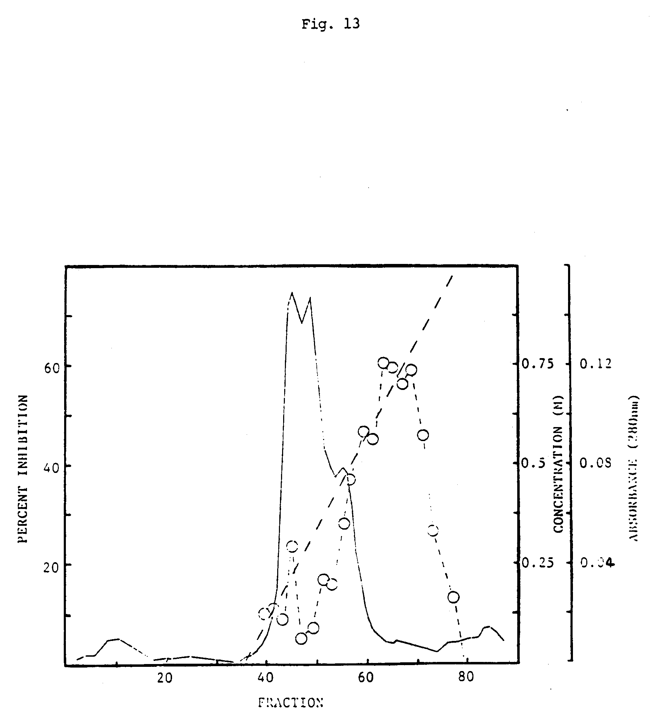

Fig. 13. Fractionation of TGI by Cation Exchange Chromatography at 4°C

CM-TRISACRYL was prepared as described in Fig. 9 with the exception that the final equilibration buffer was 20 mM ammonium acetate, pH 4.5. Protein extract (9.9 mg) prepared as above was dialyzed extensively against 20 mM ammonium acetate (pH 4.5) and applied to a 15 ml (1.5 x 8.5 cm) column of CM-TRISACRYL in 20 mM ammonium acetate (pH 4.5). Absorbance at 280 nM (-) and inhibitory activity

( ▽- -▽--▽ )

against A549 human lung carcinoma cells were determined as described in Fig. 11. The volume of the linear 0-1.0 M NaCl gradient was 150 ml. Fraction volume was 3.7 ml.

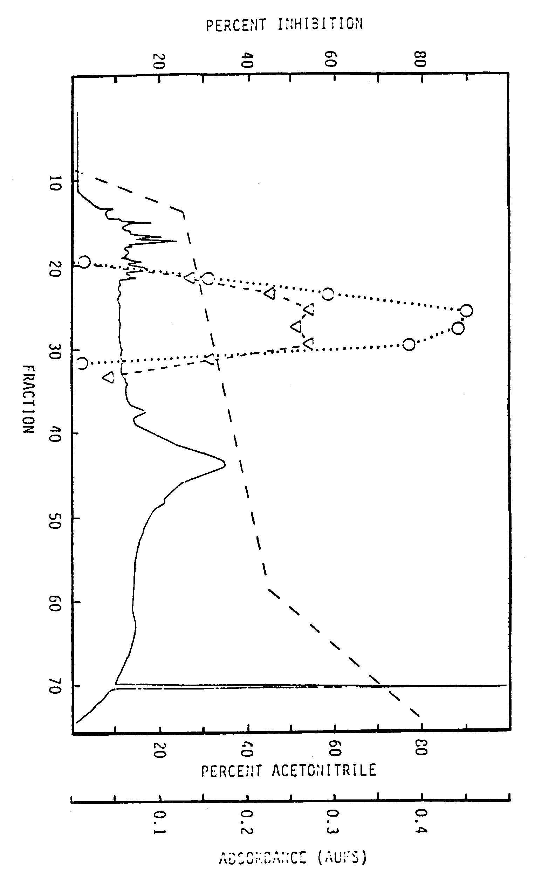

Figure 14. Reverse phase high performance liquid chromatography (HPLC) of active fractions from cation exchange chromatography

Fractions 59 thru 78 derived from cation exchange chromatography on CM-TRISACRYL of human umbilical cord described in Fig. 13 were pooled, lyophilized, and resuspended in 10 ml of 0.05% trifluoroacetic acid (TFA). A total of twenty percent of dialyzed material containing 240 ug protein was injected in three separate injections through a Water's U6K injector equipped with a 2 ml sample loop. The sample was then loaded onto a uBONDAPAK $C_{18}$ column (0.39 x 30 cm) (Waters 27324). The flow rate was 1 ml/min and the effluent monitored at 206 nm

( ——————— )

with a Waters u.v. detector (Waters Model 481) at a sensitivity of 0.5 AUFS. Elution was achieved with a linear 5-min gradient from 0-25% of increasing concentrations of acetonitrile containing 0.05% TFA, followed by a linear 15-min gradient of 25-45% acetonitrile containing 0.05% TFA, followed by a linear 15-min gradient of 45-80% acetonitrile containing 0.05% TFA, followed by a linear 5-min gradient of 80-100% acetonitrile containing 0.05% TFA. A SuperRac (LKB 2211) was used to collect 1 ml fractions. Five hundred microliter aliquots of every other fraction were transferred to 12 x 75 mm polystyrene tubes (Falcon 2058) containing 50 ul of 1.0 M acetic acid and 50 ug of bovine serum albumin (Sigma A0281) and assayed for TGI activity as described under Materials and Methods. Inhibition of A549 human lung carcinoma cells is shown by open triangles and of mink lung (CCL64) cells by open circles. The solvent gradient is shown by large dashes

(— — —) .

Detail Description of the Invention

The present invention provides a composition of matter designated tissue-derived growth inhibitor-1 (TGI-1) which comprises at least one polypeptide having the property of inhibiting the growth of human tumor cells and of an established mink lung cell line (CCL64) but not the growth of normal human foreskin fibroblasts and having an apparent molecular weight in the range from 5,000-16,000 daltons.

TGI-1 is derived from an acidified ethanol extract from human tissue which comprises a plurality of acidic polypeptides, each of which has a molecular weight of less than 20,000 daltons and each of which has the property of inhibiting the growth of human tumor cells and of an established mink lung cell line (CCL 64) while stimulating the growth of normal human foreskin fibroblasts, the inhibitory activity against human tumor cell growth not being destroyed upon increasing the temperature of the acidified, ethanol extract to 100° C for 3 minutes or upon adding acetic acid until the acidified, ethanol extract is up to 1.0 molar in acetic acid and the inhibitory activity being enhanced when the acidified, ethanol extract is prepared at 4° C rather than at 23° C.

Also, TGI-1 is recoverable as a defined activity on high performance liquid chromatography of an acidified, ethanol extract with a linear gradient of acetonitrile containing 0.05% trifluoroacetic acid at 26-34% acetonitrile.

Further, TGI-1 is recoverable as a defined activity on high performance liquid chromatography of the acidified, ethanol extract with a linear gradient of 2-propanol containing 0.05% trifluoroacetic acid at 17-23% 2-propanol.

In a preferred embodiment, the human tissue is human umbilical cord, although other tissues, e.g. human placenta, may be used.

The present invention further provides a composition of matter designated tissue-derived growth inhibitor-2 (TGI-2) which comprises at least one polypeptide having the property of inhibiting the growth of human tumor cells and of an established mink lung cell line (CCL 64) but not the growth of normal human foreskin fibroblasts and having an apparent molecular weight in the range from 5,000-16,000 daltons.

TGI-2 is recoverable as a defined activity on high performance liquid chromatography of an acidified ethanol extract derived from human tissue the extract comprising a plurality of acidic polypeptides, each of which has an apparent molecular weight less than 20,000 daltons and each of which has the property of inhibiting the growth of human tumor cells and of an established mink lung cell line (CCL 64) while stimulating the growth of normal human foreskin fibroblasts, the inhibitory activity against human tumor cell growth not being destroyed upon increasing the temperature of the acidified, ethanol extract to 100° C for 3 minutes or upon adding acetic acid until the acidified, ethanol extract is up to 1.0 molar in acetic acid and the inhibitory activity being enhanced when the acidified, ethanol extract is prepared at 4° C rather than at 23° C.

Moreover, TGI-2 is recoverable as a defined activity on high performance liquid chromatography with a linear gradient of acetonitrile containing 0.05% trifluoroacetic acid at 35-39% acetonitrile.

Further, TGI-2 is recoverable as a defined activity on high performance liquid chromatography of acidified, ethanol extract with a linear gradient of 2-propanol containing 0.05% trifluoroacetic acid at 23-27% 2-propanol.

The invention also provides a pharmaceutical composition comprising an effective amount of the composition of matter designated TGI-1 and a suitable pharmaceutical carrier.

The invention further provides a pharmaceutical composition comprising an effective amount of the composition of matter designated TGI-2 and a suitable pharmaceutical carrier.

Additionally, this invention provides a method for preparing a composition of matter designated tissue-derived growth inhibitor-1 (TGI-1). This method comprises first preparing an acidified, ethanol extract and then recovering TGI-1 from the acidified, ethanol extract as a defined activity by high performance liquid chromatography of the acidified, ethanol extract with first i) a linear gradient of acetonitrile containing 0.05% trifluoroacetic acid at 26-34% acetonitrile and then ii) a linear gradient of 2-propanol containing 0.05% trifluoroacetic acid at 17-23% 2-propanol.

The acidified, ethanol extract is prepared from human tissue and comprises a plurality of polypeptides, each of which has a molecular weight less than 20,000 daltons and each of which has the property of inhibiting the growth of human tumor cells and of an established mink lung cell line (CCL64) while stimulating the growth of normal human foreskin fibroblasts.

The method of preparing an acidified, ethanol extract comprises, under suitable conditions, the following steps: a) treating the tissue to produce lysed cells and removing the solubilized proteins derived from the cells; b) recovering the solubilized proteins; c) separately recovering from the solubilized proteins polypeptides having an apparent molecular weight less than 20,000 daltons; d) assaying the separately recovered polypeptides to identify those which inhibit the growth of human tumor cells and inhibit the growth of an established mink lung cell line (CCL64) while enhancing the growth of normal human foreskin fibroblasts; and e) recovering an acidified, ethanol extract containing the polypeptides so identified.

In a presently preferred embodiment, recovering the solubilized proteins comprises separating the solubilized proteins from the lysed cells, e.g., by centrifugation, raising the pH to 5.0, e.g., by adding ammonium hydroxide, removing the precipitate e.g. by centrifugation, precipitating the proteins from the supernatant, e.g. by adding an alcohol, an ether or both, separating the precipitate from the solution, e.g. by allowing the solution to stand at 4°C for 48 hours and by centrifugation, removing the organic phase from the precipitate, allowing the precipitate to dry, e.g. in a fume hood, redissolving the dried precipitate in a suitable solvent, e.g. 1 M acetic acid, and removing low molecular weight solutes from the solution, e.g. by dialysis.

In a presently preferred embodiment the separate recovery of polypeptides from the solubilized proteins comprises gel filtration chromatography of the proteins, e.g. on Bio-Gel P10 resin.

Also, this invention provides a method for preparing a composition of matter designated tissue-derived growth inhibitor-2 (TGI-2). TGI-2 comprises first preparing an acidified, ethanol extract and then recovering TGI-1 from the acidified, ethanol extract as a defined activity by high performance liquid chromatography of the acidified, ethanol extract with first i) a linear gradient of acetonitrile containing 0.05% trifluoroacetic acid at 35-37% acetonitrile and then ii) a linear gradient of 2-propanol containing 0.05% trifluoroacetic acid at 23-27% 2-propanol.

The acidified, ethanol extract is prepared from human tissue and comprises a plurality of polypeptides, each of which has a molecular weight less than 20,000 daltons and each of which has the property of inhibiting the growth of human tumor cells and of an established mink lung cell line (CCL 64) while stimulating the growth of normal human foreskin fibroblasts.

The method of preparing an acidified, ethanol extract comprises, under suitable conditions, the following steps: a) treating the tissue to produce lysed cells and removing the solubilized proteins derived from the cells; b) recovering the solubilized proteins; c) separately recovering from the solubilized proteins polypeptides having an apparent molecular weight less than 20,000 daltons; d) assaying the separately recovered polypeptides to identify those which inhibit the growth of human tumor cells and inhibit the growth of an established mink lung cell line (CCL64) and enhance the growth of normal human foreskin fibroblasts; and e) recovering an acidified, ethanol extract containing the polypeptides so identified.

Further, this invention provides a method for detecting the presence of a tumor which comprises quantitatively determining the amount of TGI-1 present in a sample from a subject and comparing the amount so determined with the amount present in a sample from a normal subject, the presence of a significantly different amount indicating the presence of a tumor.

Additionally, this invention provides a method for detecting the presence of a tumor which comprises quantitatively determining the amount of TGI-2 present in a sample from a subject and comparing the amount so determined with the amount present in a sample from a normal subject, the presence of a significantly different amount indicating the presence of a tumor.

The present invention also provides a method for detecting the presence of a tumor which comprises quantitatively determining the amount of TGI-1 and of transforming growth factor alpha (TGF-alpha) present in a sample from a subject, determining the ratio of the amount of TGI-1 present in the sample to the amount of TGF-alpha, determining the comparable ratio for a sample from a normal subject and comparing the ratio for the normal subject, a significantly different amount indicating the presence of a tumor.

This invention further provides a method for detecting the presence of a tumor which comprises quantitatively determining the amount of TGI-2 and of transforming growth factor alpha (TGF-alpha) present in a sample from a subject, determining the ratio of the amount of TGI-2 present in the sample to the amount of TGF-alpha, determining the comparable ratio for a sample from a normal subject and comparing the ratio for the normal subject, a significantly different amount indicating the presence of a tumor.

This invention is illustrated in the Experimental Details section which follows. This section is set forth to aid an understand of the invention but is not intended to, and should not be construed to, limit in any way the invention as set forth in the claims which follow.

In a presently preferred embodiment, recovering the solubilized proteins comprises separating the solubilized proteins from the lysed cells, e.g., by centrifugation, raising the pH to about 5.0, e.g., by adding ammonium hydroxide, removing the precipitate, e.g., by centrifugation, precipitating the proteins from the supernatant, e.g., by adding an alcohol, an ether, or both, removing the precipitate from the solution, e.g., by allowing the solution to stand at about 4°C for about 48 hours and by centrifugation, removing the organic phase from the precipitate, allowing the precipitate to dry, e.g., in a fume hood, redissolving the dried precipitate in a suitable solvent, e.g., 1 M acetic acid, and removing low molecular weight solutes from the solution, e.g, by dialysis.

In a presently preferred embodiment the separate recovery of polypeptides from the solubilized proteins comprises gel filtration chromatography of the proteins, e.g., on Bio-Gel P-10 resin.

In a presently preferred embodiment, assaying the polypeptides comprises separately contacting the human tumor cells, e.g. human lung carcinoma line (A549), or an established mink lung cell line (CCL64), or normal human foreskin fibroblasts (HuF), under suitable conditions for a suitable period of time with the polypeptides so as to identify polypeptides which inhibit the growth of the human tumor cells or inhibit the growth of the established mink lung cell line (CCl64) or which stimulate the growth of normal human foreskin fibroblasts.

A method for preparing a composition of matter designated tissue-derived growth inhibitor-1 (TGI-1) which comprises first preparing the acidified, ethanol extract and then recovering TGI-1 from the acidified, ethanol extract as a defined activity by high performance liquid chromatography, e.g., reversed phase HPLC of the acidified, ethanol extract with first i) a linear gradient of acetonitrile containing 0.05% trifluroacetic acid at about 26-34% acetonitrile and then ii) a linear gradient of 2-propanol containing 0.05% trifluroacetic acid at about 17-23% 2-propanol.

A method for preparing a composition of matter designated tissue-derived growth inhibitor (TGI) which comprises first preparing an acidified, ethanol extract and then recovering TGI from the acidified, ethanol extract as a defined activity on high performance liquid chromatography, e.g. reverse phase HPLC of the acidified, ethanol extract with a linear gradient of acetonitrile containing 0.05% trifluroacetic acid at about 28-34% acetonitrile, or as a single peak of activity from a cation exchange resin, e.g., CM-TRISACRYL resin when eluted with a linear NaCl gradient at about 0.6-0.7 M NaCl.

A method for preparing a composition of matter designated tissue-derived growth inhibitor-2 (TGI-2) which comprises first preparing the acidified, ethanol extract and then recovering TGI-2 from the acidified, ethanol extract as a defined activity by high performance liquid chromatography, e.g., reverse phase HPLC of the acidified, ethanol extract with first i) a linear gradient of acetonitrile containing 0.05% trifluroacetic acid at about 35-39% acetonitrile and then ii) a linear gradient of 2-propanol containing 0.05% trifluroacetic acid at about 23-27% 2-propanol.

A method of preparing a heterogeneous population of polypeptides designated CM-I, CM-II, CM-III and CM-IV which comprises first preparing the acidified, ethanol extract and then recovering the heterogenous population of polypeptides from the acidified, ethanol extract by ion exchange chromatography, e.g. cation exchange chromatography.

A method for detecting the presence of a tumor is disclosed. The method comprises quantitatively determining the amount of TGI-1, TGI, TGI-2 or the heterogeneous population of polypeptides designated CM-I, CM-II, CM-III and CM-IV present in a sample, e.g., blood, amniotic fluid, peritoneal fluid, ascites fluid, cerebrospinal fluid or urine, from a subject and comparing the amount so determined with the amount present in a sample from a normal subject, the presence of a significantly different amount, e.g. a significantly higher amount, indicating the presence of a tumor.

Another method for detecting the presence of a tumor is disclosed. The method comprises separately quantitatively determining both the amount of TGI-1, TGI, TGI-2 or the heterogeneous population of polypeptides designated CM-I, CM-II, CM-III and CM-IV and of transforming growth factor alpha (TGF-alpha) present in a sample from a subject, determining the ratio of the amount of TGI-1, TGI, TGI-2 or the heterogeneous population of polypeptides designated CM-I, CM-II, CM-III and CM-IV present in the sample to the amount of TGF-alpha present in the sample from a subject, determining the ratio of the amount of TGI-1, TGI, TGI-2 or the heterogeneous population of polypeptides designated CM-I, CM-II, CM-III and CM-IV present in the sample, determining the comparable ratio for a sample from a normal subject and comparing the ratio for the sample from the subject to the ratio for the sample from the normal subject, a significant variation in the ratio indicating the presence of a tumor.

A method for typing tumors is disclosed which comprises determining for a sample from a subject with a tumor the presence of one or more TGI-1, TGI, TGI-2 or the heterogeneous population of polypeptides designated CM-I, CM-II, CM-III and CM-IV, the presence or absence of a specific combination thereof, e.g., TGI and CM-II or TGI-1 and TGI-2 being indicative of a specific tumor type, e.g., a melanoma or a carcinoma.

Finally, a method for typing tumors is disclosed which comprises quantitatively determining for a sample from a subject with a tumor the amount of each of TGI-1, TGI, TGI-2 or the heterogeneous population of polypeptides designated CM-I, CM-II, CM-III and CM-IV present in the sample, the presence of specific amounts or relative amounts thereof, e.g, a significant increase in the amount of TGI or a significant variation in a ratio such as the ratio of TGI-1 to CM-II.

EXPERIMENTAL DETAILS

First Series of Experiments

Materials and Methods

Under First Series of Experiments, "TGI activity" refers to the activity of TGI-1, TGI-2, CM-I, CM-II, CM-III and CM-IV unless otherwise noted.

Isolation of Tissue-Derived Tumor Growth Inhibitors (TGIs) From Tissue Extracts

Human umbilical cord or placenta tissues were extracted using a modification of the acid/ethanol extraction procedure described by Davoren et al (Biochem. Biophys. Acta. 63:150 (1962) and Roberts et al, Proc. Natl. Acad. Sci. USA. 77:3494 (1980).

The buffer for extraction consisted of 375 ml of 95% (v/v) ethanol (punctilious, 190 proof, U.S. Industrial Chemicals, #UN1170), 7.5 ml of concentrated HCl, 33 mg of phenylmethylsulfonyl fluoride (PMSF) (Sigma P-7627) and 1 ml of Aprotinin (Sigma A6012 with 19.8 Trypsin inhibitor units per ml in 0.9% NaCl and 0.9% benzyl alcohol) mixed with 192 ml of distilled water at 4°C. Four hundred to six hundred grams of frozen human umbilical cords or placentas (Advanced Biotechnologies) (stored at -80°C) were thawed at 4°C for six hours. The thawed tissue was placed in a 4°C chilled Cuisinart food processor (Model DLC-7-PRO) and suspended in 200 ml of 4°C extraction buffer. The suspended tissue was homogenized by the food processor. After the first minute of homogenization, the suspension became creamy white. Another 200 ml of 4°C extraction buffer was added to this white suspension. The suspension changed to a dark coffee brown color. The tissue suspension was homogenized for a total of 10 min. at 4°C. Extraction buffer was added to this homogenized tissue mixture to a final volume of 6 ml per gram of tissue homogenate.

The homogenized tissue suspension was transferred to a large 4 liter beaker with a 3 inch stir bar and stirred at half of the maximum stirring capacity of a Lab-line Multimagnestir multi-mixer, Model #1278. After overnight extraction with stirring at 4°C, the homogenate was transferred to 1 liter centrifuge bottles (Sorvall) and centrifuged at 3500 rpm (RCF = 350) for 30 minutes at 4°C in a Sorvall RC-3B centrifuge equipped with a Sorval H-6000A rotor. The supernatant was transferred to a large 4 liter beaker and adjusted to pH 5.0 with the slow addition of concentrated ammonium hydroxide. With increasing pH, the color of the supernatant changed from brown to an orange solution. The solution was precipitated following the addition of 2.0 M ammonium acetate, pH 5.2, added at an amount of 1% of the total volume. This precipitate was removed following centrifugation at 4500 rpm (RCF = 5900) for 4 hours in a Sorvall RC-3B at 4°C. The supernatant was transferred to large 6 liter flasks to which four volumes of anhydrous ether (-20°C) (Baker 9244-3) and two volumes of 95% ethanol (4°C) were added. The mixture was allowed to stand undisturbed at -220°C for 48 hours to allow the resulting precipitate to settle.

At the end of the 48 hour precipitation, the etherized material was brought to ambient temperature in a fume-hood. Warming of the acidified, ethanol extract to ambient temperature enhances the aggregation of the precipitate. The clear organic phase of ether and ethanol was removed by a water aspirator and the precipitate was left in the fume hood for several hours to allow the residual organic phase to evaporate. The "dried" precipitate was dissolved in 1.0 M acetic acid and dialyzed estensively against 1.0 M acetic acid (Baker #9507-5) using dialysis membranes with a molecular cutoff of 3500 (Spectropor 3, Spectrum Medical Industries, Los Angeles, CA). The dialyzed acidified ethanol extract was lyophilized in 250 ml Corning conical centrifuge tubes (Corning 25350) and stored as crude acidified, ethanol extract.

An alternative procedure for precipitating TGIs from the acidified, ethanol extract replaces the additi on of four volumes of ether and two of ethanol with the addition of only the two volumes of ethanol at 4°C. The advantage of eliminating ether from the acidified, ethanol extract precipitation step was the elimination of a step requiring the use of a highly flammable solvent which makes the procedure and any scale-up of the processing of large amounts of materials difficult.

Gel Filtration Chromatography

Lyophilized crude acidified, ethanol extract was resuspended in 1.0 M acetic acid (10-30 mg/ml) and clarified by centrifugation at 3500 rpm for 30 min at 4°C in a Sorval RC-3B centrifuge equipped with a Sorvall H-6000A rotor before sample application to the column. Sample volumes of one hundred to 150 ml were chromatographed on Bio-Gel P10, 100-200 mesh (Bio-Rad; 150-1040) in 1.0 M acetic acid at either 23° or 4°C.

The column (14 x 100 cm) (Amicon; #86012) contained 13.8 liters of equilibrated and degassed Bio-Gel P 10 in 1.0 M acetic acid at either 23°C or 4°C. The void volume was determined by the addition of 50 ml of blue dextran (Sigma #D5751) at 2 mg/ml in 1.0 M acetic acid. After calibration, the column was "conditioned" with 100 ml of bovine serum albumin (Sigma #A-4503) at 100 mg/ml in 1.0 M acetic acid followed by extensive washing with 1.0 M acetic acid.

Following sample application, 1 liter fractions were collected using a SuperRac (LKB 2211) equipped with a type C collection rack, at a flow rate of 7 ml/min into 2 liter plastic tissue culture roller bottles (Falcon; 3207). Fractions were monitored by a Uvicord S (LKB 2138) at 280 nm set at an absorbance range of 2.0 AUFS and recorded by a single channel chart recorder (LKB 2210). One ml aliquots were removed from each fraction, lyophilized and assayed for TGI activity as described. The remainder of each fraction was lyophilized in 2 liter lyophilization jars Virtis #6503-2050) using a Virtis freeze-model 24.

## High performance liquid chromatography (HPLC)

Individual fractions containing TGI activity from the Bio-Gel P-10 column were lyophilized and resuspended in 1 to 10 ml of 0.05% trifluoroacetic acid (TFA) (Pierce #28901) depending upon the amount of protein in each fraction. Water used for HPLC was generated using a Milli-Q water purification system. Starting buffer in all HPLC chromatography runs consisted of Milli-Q water containing 0.05% TFA. Prior to injection, the sample was centrifuged in a Beckman tabletop centrifuge (Beckman TJ-6) at 3000 rpm for 20 min to remove insoluble material. The supernatant was injected into either a Waters uBondapak analytical $C_{18}$ column (0.39 x 30 cm) (Waters PN27324) or semipreparative column (0.78 x 30 cm) (Waters PN84176) as specified in individual experiments. A Waters automatated gradient controller (Waters Model 510) was utilized for column elution monitored by variable wavelength u.v. detectors (Waters Lambda-Max, Model 481) set at 206 nm. The solvent used for elution was either acetonitrile (Baker 9017-3) or 2-propanol (Fisher, A452) containing 0.05% TFA. Fractions were collected by a SuperRac (LKB 2211) equipped with a type B collection rack into siliconized (Pierce, Aquasil #42799) 13 x 100 mm or 16 x 100 mm test tubes. Aliquots from each collected fraction were assayed for TGI activitiy as described below.

## Ion exchange chromatography

Both the lyophilized material from the acidified, ethanol and ether extractions and various lyophilized fractions derived from the Bio-Gel P-10 gel filtration chromatography were separately subjected to ion exchange chromatography. CM, SP, and DEAE-TRISACRYL (LKB) ion exchange resins were used in these procedures. The samples for chromatography were diluted to a final concentration of approximately 20 mg/ml in 1.0 M acetic acid. The samples were dialyzed at 4°C until both the pH and conductivity were equal to the starting (equilibration) buffer. All ion exchange chromatographic procedures were performed at 4°C.

## a. Chromatography using CM- and SP-TRISACRYL ion exchange results.

The resins, as aqueous suspensions, were suspended in an equal volume of 0.1 M ammonium acetate, pH 4.0, containing 1.0 M NaCl. The resin was allowed to equilibrate for at least 3 hours and was degassed at 4°C. Twenty ml of resin was packed into a 1.6 x 20 cm column (Pharmacia; #19-0362-01) and washed with 2 column volumes of 1.0 ammonium acetate, pH 4.0, followed by 0.01 M ammonium acetate, pH 4.0. The column was washed until the effluent exactly matched the conductivity of the equilibrating buffer (i.e., 0.01M ammonium acetate, Fisher A637), pH 4.0. The sample was applied to the resin (1 gm/20 ml resin) at a flow rate of 1 ml/min, the column was washed with equilibration buffer until the optical density leveled (e.g., approaching zero optical density) and 200 ml of an ascending molarity linear gradient (Pharmacia gradient mixer GM-1, #19-0495-01) was applied through a column flow adaptor of concentrations 0.01 to 1.0 M ammonium acetate, pH 4.0. In certain experiments, a second gradient was applied to the same column. This second gradient ranged from 1.0 M ammonium acetate, pH 4.0, to 50% acetonitrile in 1.0 M ammonium acetate, pH 4.0. Two ml fractions were collected in polystyrene tubes, 13 x 100mm, (Columbia Diagnostics; B2564) in a SuperRac Fraction collector (LKB 2211), equipped with an A type collection rack. All column chromatography was performed with the aid of a Uvicord S with a 280 nm filter (LKB 2138) and

14

a single channel recorder (LKB 2210). Fractions were aliquoted based upon optical density ranging from 100ul to 1 ml, and assayed for TGI activity.

### b. Chromatography using DEAE-TRISACRYL

The chromatographic resin preparation and procedure was performed exactly as described for CM- and SP-TRISACRYL chromatography, except the equilibration buffer used was 0.1 M ammonium acetate, pH 6.0, the gradient elution ranged from 0.1 M to 1.0 M ammonium acetate, pH 6.0, and the sample was equilibrated in the above mentioned equilibration buffer.

### Monolayer assay for TGI activity

Test cells were sub-cultured on 96-well tissue culture plates (Nunc 167008) in 50 ul of Dulbecco's modified Eagle's medium (Whittaker M.A. Bioproducts 12-6143) containing 10% fetal bovine serum (Whittaker M.A. Bioproducts 14-501B), 2% L-glutamine (Whittaker M.A. Bioproducts 17-605-A), 1% penicillin and 1% streptomycin. Human lung carcinoma cells, A549, and normal human fibroblasts (HuF) required a seeding density of $5 \times 10^3$ cells per well. Mink cells (ATCC: CCL64) required a seeding density of $4.5 \times 10^3$ cells per well.

Aliquots from column fractions to be assayed for TGI activity were transferred to sterile 12 x 75 mm tubes (Falcon 2058) containing 50 of ul 1mg/ml solution of bovine serum albumin (BSA; Sigma A-6003) in 1 M acetic acid and lyophilized. Immediatetly prior to the assay, the lyophilized sample was resuspended in 400 ul, for each cell type tested. One hundred ul aliquots of the resuspended sample were added to wells containing test cells. Each sample was assayed in triplicate. The cells were incubated for 72 hours at 37° in a humidified 5% $CO_2$/95% air atmosphere. At the end of the incubation period, each well was pulsed with 100 ul of complete medium containing 1 uCi/ml 5-[$^{125}$I]Iodo-2'deoxyuridine ([125]IUdR) (New England Nuclear; NEX-072) for 24 hours. The monolayers were washed once with wash buffer A (Dulbecco's phosphate buffered saline, with 10 mM $MgCl_2$, containing 1 mg/ml BSA, pH 6.8), fixed for 10 minutes in methanol (Fisher A452), and air dried for 15 minutes. The [125]IUdR incorporated by the cells was solubilized with 200 ul of 1.0 N NaOH and the plates incubated for 20 minutes at 60°C. Solubilized [125]IUdR was collected using the Titertek Supernatant Collection System (Skatron Inc., 7072). The amount of cell growth is approximated by the extent of [125]IUdR incorporated into the DNA of cells in the log phase of growth. Before the assay was harvested each well was observed using a Zeiss inverted microscope to visually note the amount of cell growth. Inhibition or stimulation of growth was expressed as a ratio of [125]IUdR incorporated by test cells (e.g. human tumor cells) containing the test aliquots relative to [125]IUdR incorporated by the untreated control cells. The inhibition or stimulation observed by microscopic examination of treated cells corresponded well with decreased or increased incorporation of [125]IUdR, respectively.

### Characterization of TGI activities

### a. Heat Treatment

One ml aliquots from fractions 2, 4, and 6 obtained from gel filtration chromatography on Bio-Gel P-10, were lyophilized in 12 x 75 mm polystyrene tubes (Falcon 2034) and resuspended in 1 ml of 1.0 M acetic acid. The samples were heated for 3 minutes in a boiling water bath, lyophilized, and assayed for TGI activity as described above.

### b. Treatment with Dithiothreitol (DTT)

Three ml aliquots from fraction 2, 4 and 6 obtained from gel filtration chromatography on Bio-Gel P-10, were lyophilized in 17 x 125 mm polystyrene screw cap tubes (Columbia Diagnostics #2570). Treated samples received 200 ul of 0.1 M ammonium bicarbonate containing a final concentration 0.1 M DTT (Calbiochem #233-153), pH 7.4. Control samples contained 200 ul 0.1 M ammonium bicarbonate (Fisher, A643), pH 7.4. The samples were incubated for one hour at 23°C, diluted to 1 ml with 1.0 M acetic acid, and dialyzed in 18 mm dialysis membranes with molecular weight cutoff of 3,500 daltons (Spectropor 3, Spectrum Medical Industries), against four changes of 4 liters of 0.1 M acetic acid. The samples were lyophilized in 12 x 75 mm polystyrene tubes (Falcon #2304) and assayed for TGI activity as describe above.

SDS-Polyacrylamide slab gel electrophoresis

Aliquots from samples from each chromatographic procedure were lyophilized for electrophoresis. Samples were diluted in 80 ul of sample buffer containing 0.1 M Tris-HCl (Sigma; T-1503), pH 6.8, 15% glycerol (Kodak; 114-9939), 2% sodium dodecyl sulfate (SDS) Bio-Rad; 116-0302), and 5% 2-mercapto-ethanol (Bio-Rad; 161-0710), and electrophoressed on a 5-20% acrylamide monomer gradient essentially as described (Laemmli, U.K. (1970) Nature 227, 680-685). The samples were boiled for 2 minutes prior to application to a 1.5 mm wide slab gel in a Bio-Rad Model 155 Vertical Electrophoresis Cell (Bio-Rad 165-1420) under contant current at 30 mA per gel for 4 hours (Hoeffer power supply; PS 1200 DC) at 9°C. Constant temperature was maintained by a water bath circulator (Haake, A81). Gels were stained with 0.5% Coomassie Blue R250 (Bio-Rad #16-0400) in 5.7% acetic acid and 47% methanol overnight and destained in the same solution without stain. Specific gels demonstrating low concentrations of proteins were restained by a silver technique as described by Merril (Merril, C.R., Goldman, D., Sedman, S. and Ebert, M.H. (1981) 211:1437-1438), (Bio-Rad silver staining kit; #161-0443).

Results

Comparison of TGI activities from gel filtration chromatography on Bio-Gel P10 at room temperature and at 4°C. The growth inhibitory activity derived from acidified, ethanol extracts of human umbilical cords eluted by gel filtration chromatography using Bio-Gel P.10 resin with apparent molecular weights ranging from 5,000-16,000 daltons. Occasionally, another peak of activity has been observed at molecular weights ranging from 3000-5000 daltons. The molecular weight calculations are based on the elution profiles of molecular weight standards (i.e., carbonic anhydrase - 29,000; RNase - 14,400; insulin - 6,000) chromatographed on 1 liter of resin in a column of 4 x 100 cm. The elution profile derived from the column and from the large 14 x 100 cm column were superimposable. Acidified, ethanol extracts from human placenta identically chromatographed demonstrated elution profiles very similar to the umbilical cord extracts.

Fractions 1 to 3 from the umbilical cord acidified, ethanol extract are a very intense brown color; the color gradually disappears as the fractions progress. Fortunately, although tumor cell growth inhibitory activity (TGI) eluted in fractions 1, 2, and 3 containing the highest protein concentrations, the majority of activity extends past the observed protein peaks as is clearly demonstrated in Figures 1 and 2. Extracts from human placental material showed a greater overlap of TGI with the major protein peaks than was observed with material from human umbilical cords (data not shown). Aliquots of identical volumes from gel filtration chromatography electrophoresed by SDS-PAGE on a 5-20% polyacrylamide gradient also illustrated that by fraction 4, considerably less protein is found than in fractions 1 to 3. In fractions 5 an 6, major protein bands of 5,600 and 14,000 band are observed and by fraction 7 very little protein r emains, although inhibitory activity extends into fraction 10 as shown in Figure 2. The obvious advantage of the majority of activity eluting in regions of less protein is that it facilitates further purification of TGIs.

A comparison of Bio-Gel P-10 chromatograms performed at room temperature and 4°C, illustrated in Figures 1 and 2, respectively, clearly indicate that inhibitory activity is better preserved at 4°C. At 23°C, no activity is observed past fraction 6 (Figure 1), while at 4°C, activity is extended for 4 more fractions to fraction 10. Most importantly, the net amount of activity recovered is at least two-fold higher when extracts are chromatographed at 4°C, since 80% or more TGI activity is obtained in 7 fractions at 4°C (Figure 2) and in only 3 fractions at 23°C. This was not due to a concentration of the same quantity of activity eluting in 3 fractions - (23°C) rather then being spread over 7 fractions - (4°C), but apparently to actual increase in the yield of TGI activity. One ml aliquots of fraction 5 from both columns separately and dilutions of these fractions to 1/5 to 1/125 were tested on both the human lung adenocarcinoma (A549) and mink lung cells (CCL64) (Table 1). The TGI activity of the undiluted fraction was 2-fold higher in the fraction 5 obtained from chromatography at 4°C. Moreover, a 25-fold dilution of fraction 5 from chromatography at 4°C continued to yield maximum TGI activity against the human tumor cell line. A fraction of equivalent dilution from chromatography at 23°C showed no detectable activity. A similar observation was made with the mink cell line. This information was not based on activities observed in Figures 1 and 2 but from two separate columns which demonstrated equivalent TGI activities in their respective fifth fraction.

Comparison of the effects of TGIs on normal human fibroblasts (HuFs) and transformed human lung carcinoma cells (A549). Aliquots of fractions obtained from human umbilical cord acidified, ethanol extracts chromatographed on a Bio-Gel P-10 resin, (4°C), were tested for TGI activity on human normal and transformed cells as described in Materials and Methods. As illustrated in Figure 3, TGI activity against human A549 cells (open triangles) ranged from fractions 3 to 12, while these same fractions induced as much as an 85% increase in growth stimulation of the normal human fibroblasts. Thus, the inhibitory activity

16

is specific for human tumor cells. This observed inhibitory activity is not due to cytotoxicity, as demonstrated by light microscopic studies and indirectly by its stimulatory effect on normal human fibroblasts. The TGI's have previously been tested on "normal" epithelial derived cells and simlar results were observed.

## TABLE 1

## EFFECT OF TEMPERATURE ON THE RECOVERY
## OF TGI ACTIVITY FROM GEL FILTRATION CHROMATOGRAPHY

| | PERCENT INHIBITION OF THE TEST CELL | |
|---|---|---|
| TEMPERATURE OF COLUMN RUN | $4^{\circ}C$ | $23^{\circ}C$ |
| **TEST CELL LINE** | | |
| A549 (Human Carcinoma) | | |
| Undiluted | 57 | 30 |
| 1/5 | 62 | 25 |
| 1/25 | 54 | 0 |
| 1/125 | 15 | 7 |
| Mink lung (CCl 64) | | |
| Undiluted | 91 | 43 |
| 1/5 | 90 | 13 |
| 1/25 | 70 | 9 |
| 1/125 | 31 | 2 |

One ml aliquots from gel filtration on fraction 5 (Figures 1 & 2) containing 120 micrograms were used to assay TGI activity.

High performance liquid chromatography (HPLC). TGIs from acid ethanol extracts of human umiblical cords partially purified by gel filtration on a Bio-Gel P-10 column followed by further purification using reverse phase HPLC (uBondapak $C_{18}$ resin) inhibited the growth of both A549 human carcinoma and an established mink lung cell line, CCL 64, but did not inhibit the growth of normal human fibroblasts. Figure 4 illustrates an elution profile of the TGI activity obtained by HPLC using a linear acetonitrile gradient of lyophilized fraction 4 (19.8 mg/3ml 0.05% trifluroacetic acid) derived from the Bio-Gel P-10 chromatographic step.

Evidence of two distinct peaks of growth inhibitory activities against both the A549 human carcinoma and the mink cells were observed. The fractions eluting between 28-34% (fractions 13-22) acetonitrile and 35-39% (fractions 25-31) acetonitrile were pooled separately and rechromatographed on a $C_{18}$ uBondapak column using a linear gradient of 2-propanol.

The first peak of TGI activity was gnated TGI-1 and the second TGI-2. Figure 5 demonstrates the elution profile and TGI activity of TGI-1 (Figure 4). The concentration of injected material was 1.5 mg/1.5 ml of 0.05% trifluoroacetic acid (TFA). TGI-1 activity elutes between 17-23% using a linear gradient of 2-propanol. Similarly, Figure 6 indicates that TGI-2 (0.8 mg/1.8 ml 0.05% TFA) (Figure 5) rechromatographed between 23-27% (fraction 17-23) using a linear gradient of 2-propanol. The TGI activity presented in Figures 4 and 5 are consistently 20% higher against the mink cells than against the A549 human carcinoma cells.

Another total fraction from the Bio-Gel P-10 chromatographic step, fraction 5 (see Figure 3), was chromatographed by HPLC using a linear acetonitrile gradient (Figure 7). Fraction 5, which was a later fraction than the material used in Figure 4 (fraction 4), predominantly contained inhibitory activity eluting as TGI-1. In Figure 7, TGI-1 eluted between 29-34% acetonitrile. Growth inhibitory activity was 35% higher against the mink cells than the A549 human carcinoma cells. The majority of TGI activity has now been separated from the peaks of protein. The active fractions (14-25) were pooled, lyophilized, and rechromatographed by reverse phase HPLC using a linear gradient of 2-propanol (Figure 8). Evidence of differential growth inhibitory activity was observed. The TGI activities were separated into a growth inhibitory activity specific for mink cells eluting at a peak of activity of 23% 2-propanol (fractions 22-25) and one specific for the A549 human carcinoma cells at 26% 2-propanol (fractions 25-26). Therefore, a fraction eluting later from gel filtration chromatography that has less contaminating proteins [lower absorbance at 280 nm (Figure 2) and less protein by gel electrophoresis] has two different cell-specific inhibitory activities.

Acid ethanol extracts of human placenta contained TGI activities which, following a gel filtration chromatographic step, also eluted between 26-34% acetonitrile of a $C_{18}$ column using a linear acetonitrile gradient containing 0.05% TFA.

Ion exchange chromatography One gram of a lyophilyzed acidified, ethanol extract of human umbilical cords was directly subjected to ion exchange chromatography on CM-TRISACRYL in 0.01 M ammonium acetate, pH 4.0. A linear gradient was applied from 0.01 to 1.0 M ammonium acetate, pH 4.0. Figure 9 demonstrates at least 4 separate TGI activities designated CM-I, CM-II, CM-II, and CM-IV. CM-I was presently inhibited only the A549 human carcinoma cells at 60% inhibition (Table 2). CM peaks II and III have similar levels of growth inhibiting activity against both A549 human carcinoma (80 and 63%, respectively) and mink cells (61 and 76%, respectively). The last peak of activity (CM-IV) demonstrates a specificity in activity against mink (i.e. mink cells were more inhibited (95%) than were the A549 human carcinoma cells (69%)). CM-I was not retained and CM-II was slightly retarded by the negatively charged resin since they both were eluted before the gradient was started by 0.01 M ammonium acetate, pH 4.0.

Although all the proteins that have inhibitory activity are acidic proteins, since they are soluble at pH 4.0 and bind to a negatively charged resin, peaks CM-III and IV are probably slightly more basic since they bind more tightly to the CM-TRISACRYL resin (eluting at greater than 0.5M ammonium acetate). This is substantiated by the fact that no TGI activity was retained by a postively charged resin (i.e. DEAE-TRISACRYL) (data not shown). The more acidic inhibitory factors appear to be more specific for the A549 human carcinoma cells in their respective activities. These 4 peaks of TGI activities (CM-I, CM-II, CM-III, and CM-IV) have been repeatedly observed (6 separate chromatographic procedures with CM-TRISACRYL). To ensure that the TGI activities observed in CM-III and CM-IV would not yield material that could be eluted earlier from the column, and also to provide support for the notion that each peak of activity is a separate entity, material from

## TABLE 2

### TGI ACTIVITY FROM CATION EXCHANGE CHROMATOGRAPHY

| PEAK OF TGI ACTIVITY | PERCENT INHIBITION OF THE TEST CELL | |
|---|---|---|
| | A549 | Mink |
| CM I | 60 | 0 |
| CM II | 80 | 61 |
| CM III | 63 | 76 |
| CM IV | 69 | 95 |

Protein concentrations for the fractions tested for TGI activity ranged from 15-300 ug.

CM-III and CM-IV was pooled, lyophilized, and rechromatographed using CM-TRISACRYL under the same conditions as the column from which it was derived. CM-III and CM-IV eluted (greater than 0.5 M ammonium acetate) in exactly the same position as did the original column fractions from which they were derived (Figure 10). The higher TGI inhibitory activity against mink cells was preserved and the difference between the inhibitory activity against the two cell lines remained exactly the same at 25-30% around the peak of activity.

Physical and biological characterization of tissue derived tumor cell growth inhibitory activity (TGIs).

Fractions 2, 4 and 6 derived from gel filtration chromatography by Bio-Gel P-10 were either heat treated (Table 3) or reduced by dithiothreitol (DTT) treatment (Table 4). All fractions tested retained TGI activity following either heat or acid treatment (see Table 5). Fractions 2, 4 and 6 were found to inhibit human cancer cell growth and stimulate normal human cell growth. TGI activity in fractions 2 and 6 was decreased by treatment with DTT, while the TGI activity in fraction 4 was actually slightly increased (Table 4). This information further substantiates the existence of separate TGIs.

TABLE 3

EFFECT OF HEAT TREATMENT ON TGI ACTIVITY
OF FRACTIONS FROM GEL FILTRATION CHROMATOGRAPHY

| | A549 | | MINK | |
|---|---|---|---|---|
| COLUMN FRACTION | CONTROL PERCENT INHIBITION | AFTER HEAT TREATMENT PERCENT INHIBITION | CONTROL PERCENT INHIBITION | AFTER HEAT TREATMENT PERCENT INHIBITION |
| 2 | 16 | 32 | 54 | 68 |
| 4 | 63 | 65 | 78 | 80 |
| 6 | 70 | 63 | 82 | 71 |

Protein concentrations for the fractions tested from TGI activity ranged from 15-300 ug.

TABLE 4

EFFECT OF DDT TREATMENT ON TGI ACTIVITY
OF FRACTIONS FROM GEL FILTRATION CHROMATOGRAPHY

| | A549 | | MINK | |
|---|---|---|---|---|
| COLUMN FRACTION | CONTROL PERCENT INHIBITION | AFTER DDT TREATMENT PERCENT INHIBITION | CONTROL PERCENT INHIBITION | AFTER DDT TREATMENT PERCENT INHIBITION |
| 2 | 31 | 8 | 69 | 17 |
| 4 | 63 | 69 | 79 | 69 |
| 6 | 71 | 34 | 78 | 26 |

Protein concentrations for the fractions tested for TGI activity ranged from 5-300 ug.

## TABLE 5

## PHYSICAL AND BIOLOGICAL PROPERTIES OF TISSUE-DERIVED TUMOR CELL GROWTH INHIBITORY ACTIVITY (TGI)

### Column Fraction

|  | Fraction 2 | Fraction 4 | Fraction 6 |
|---|---|---|---|
| Stable to 1.0 M acetic acid | + | + | + |
| Stable to boiling at 100°C | + | + | + |
| Inhibits human cancer cells | + | + | + |
| Inhibits normal human cells | − | − | − |
| Inactivated by dithiothreitol | + | − | + |

Second Series of Experiments

Materials and Methods

Isolation of Tissue-Derived Tumor Growth Inhibitors (TGIs) From Tissue Extracts Depleted of Blood, Veins, and Arteries

Veins and arteries were removed from human umbilical cord tissues and the remaining tissues were extensively washed to remove blood prior to acid/ethanol extraction as described under First Series of Experiments.

The buffer for washing and homogenizing the tissue (PBS-PA) consisted of 2 liters of water containing 16 gm NaCl, 2.5 gm $Na_2HPO_4.H_2O$, 0.4 gm $NaH_2PO_4$ •$7H_2O$, 116 mg phenylmethylsulfonyl fluoride (PMSF) (Sigma P7627) and 3.3 ml Aprotinin (Sigma A6012 with 19.8 units Trypsin inhibitor per ml in 0.9% NaCl and 0.9% benzyl alcohol), adjusted to pH 7.4 with HCl and NaOH. The extraction buffer consisted of 375 ml of 95% (v/v) ethanol (punctilious, 190 proof, U.S. Industrial Chemicals, #UN1170), 7.5 ml of concentrated HCl, 33 mg of phenylmethylsulfonyl fluoride (PMSF) (Sigma P-7627) and 1 ml of Aprotinin (Sigma A6012) mixed with 192 ml of distilled water at 4°C. Eight hundred to one thousand grams of frozen human umbilical cords (Advanced Biotechnologies; stored at -80°C) were thawed by immersion in PBS-PA for two hours at 4°C. Individual umbilical cords were removed and rinsed with PBS-PA. Veins and arteries were removed from the umbilical cords by dissection at 4°C. The dissected umbilical cord was washed with fresh PBS-PA to remove residual blood and vascular debris.

The tissue was placed in a 4°C chilled Cuisinart food processor (Model DLC-7-PRO) and suspended in

21

200 ml of 4°C PBS-PA. The suspended tissue was homogenized by the food processor. After the first minute of homogenization, an additional 200 ml of 4°C PBS-PA was added. The tissue suspension was homogenized for a total of 10 min. at 4°C. The homogenate was transferred to 200 ml centrifuge bottles (Sorvall) and centrifuged at 9000 rpm (RCF = 13,000) for 5 minutes at 4°C in a Sorvall RC5B centrifuge equipped with a Sorvall GSA rotor. The supernatant fluid was removed and discarded and the pellet resuspended to the original homogenate volume with fresh PBS-PA.

The pellet was washed by repeated centrifugation and resuspension as described until the supernatant fluid was clear with no tint of red from contaminating blood or blood products. The resulting washed pellet was white. The washed pellet was resuspended in the buffer for extraction to a final volume of 6 ml per gram of original dissected tissue. The homogenate was transferred to a large 4 liter beaker with a 3 inch stir bar and stirred at half of the maximum stirring capacity of a LAB-line Multimagnestir multimixer, Model #1278. After overnight extraction with stirring at 4°C, the homogenate was transferred to 1 liter centrifuge bottles (Sorvall) and centrifuged at 3500 rpm (RCF = 3570) for 30 minutes at 4°C in a Sorvall RC-3B centrifuge equipped with a Sorvall H-6000A rotor. The supernatant was transferred to a large 4 liter beaker and adjusted to pH 5.0 with the slow addition of concentrated ammonium hydroxide. With increasing pH, the supernatant remained clear with a slight yellowish tint. A 2.0 M solution of ammonium acetate, pH 5.2, was added in an amount 1% of the total volume. Any precipitate formed by this step was removed by centrifugation at 4500 rpm (RCF = 5900) for 4 hours in a Sorvall RC-3B at 4°C. The supernatant was transferred to large 6 liter flasks to which four volumes of anhydrous ether (-20°C) (Baker #9244-3) and two volumes of 95% ethanol (4°C) were added. The mixture was allowed to stand undisturbed at -20°C for 48 hours to allow the resulting precipitate to settle.

At the end of the 48 hr precipitation, the material was brought to ambient temperature in a fumehood. Warming of the acidified, ethanol extract to ambient temperature enhances the aggregation of the precipitate. The clear organic phase of ether and ethanol was removed by a water aspirator and the precipitate remained in the fume hood for several hours to allow the residual organic phase to evaporate. A gentle stream of dried nitrogen gas over the extract accelerated the evaporation of the remaining organic solvent present with the precipitate. The "dried" precipitate was dissolved in 1.0 M acetic acid and dialyzed extensively against 1.0 M acetic acid (Baker #9507-5) using dialysis membranes with a molecular weight cutoff of 3500 (Spectropor 3, Spectrum Medical Industries, Los Angeles, CA). The dialyzed acidified extract was lyophilized in 250 ml Corning conical centrifuge tubes (Corning 25350) and stored as crude acidified, ethanol extract or dialyzed extensively against 20 mM $NH_4O_2C_2H_3$, pH 4.5.

Comparison of TGI activity in the initial acid/ethanol extract from tissue prepared as described in the First Series of Experiments with tissue prepared as described above.

The improvement in the specific activity and total recovered activity seen when the tissue was prepared as described above is shown in Table 6. The table compares the yields of protein and TGI activity from frozen umbilical cord when it was processed according to the procedures detailed in the First Series of Experiment (hereinafter "inital procedure") and when it was processed as describe above (hereinafter "modified procedure").

There are several obvious differences in the two procedures which are of importance for the subsequent purification of TGI. For example, based on the wet weight of the tissue, acidified ethanol extraction by the initial procedure resulted in the recovery of 0.33% as protein (3.3 g from 1000 g tissue) whereas only 0.015% as protein (0.05 g from 340 g tissue) was extracted when following the modified procedure. Because the yield of activity was 50% greater (3.3 x $10^6$ units) by the modified procedure than in the initial procedure (2 x $10^6$ units) from 66% less tissue (340g vs 1000g) the overall efficiency of extraction was improved. The initial procedure yielded 2000 units of TGI activity per gram of umbilical cord (wet weight). The modified procedure yielded 9700 units of TGI activity per gram of umbilical cord (wet weight). The overall efficiency of extraction was improved 5-fold by the modified procedure. Furthermore, since less protein was extracted by acidified ethanol, the volumes of ether and ethanol required to precipitate the extracted proteins

TABLE 6

Comparison of TGI Activity in the Initial Acid/Ethanol Extract from Tissue Prepared as Described in the Initial Procedure with that Prepared as Described in the Modified Procedure

| Tissue[1] (wet weight) | Procedure | Protein (extracted) | Total[2] Activity | Specific[2] Activity |
|---|---|---|---|---|
| 1000 g | initial | 3.0 g | $2 \times 10^6$ u | 0.67 u/ug protein |
| 340 g | modified | 50 mg | $3 \times 10^6$ u | 67 u/ug protein |

[1] Human umbilical cord

[2] A unit of activity is defined as that amount of material which results in 50% of the maximal inhibition seen with a given cell line, e.g., the A549 (as used for this table) cell line is maximally inhibited 60%, therefore a unit of activity is equivalent to 30% inhibition.

are less. Finally, the amounts of protein and the numbers of different proteins extracted by the modified procedure are fewer and therefore the subsequent purification procedures to be employed will require less chromatographic materials, shorter processing times and fewer steps to obtain a pure product.

Fractionation of TGI extracted using the modified procedure on the cation exchange resin CM-TRISACRYL was resolved as a single peak from the bulk of the applied protein when the bound material was eluted by a linear salt gradient from 0-1.0 M NaCl. Figure 11 shows that following application of TGI to a CM-TRISACRYL column no inhibitory activity was detectable from material not bound to the resin (i.e.,

fractions 1-24). The linear addition of increasing amounts of NaCl (- -) removed the majority of protein bound to the resin (fractions 25-38) prior to the removal of significant amounts of inhibitory activity

$$( \nabla - - - \nabla \, ,$$

fractions 39-49). The NaCl concentration most effective in removing bound TGI was approximately 0.6 M (fraction 44). Comparison of Figure 11 with Figure 10 suggests that the inhibitory activity eluted in the experiment of Figure 11 most closely corresponds to the elution of CM-III and CM-IV from the CM-TRISACRYL resin as depicted in Figure 9 since the salt concentrations (NaCl, Figure 11; $NH_4O_2C_2H_3$, Figure 10) for elution are similar (0.6 M, Figure 11; 0.6-0.7 M, Figure 10). The above information also suggests that treatment of the tissue by the modified procedure allows the preferential isolation of a single type of TGI thus improving subsequent characterization of the factor.

Another property of the TGI extracted from the tissue by the modified procedure is its failure to bind to anion exchange resin. Figure 12 shows that following adjustment of the pH to 8.0 as described in the figure legend and application of the extract (an identical amount to that used in Figure 11) to the anion exchange resin DEAE-TRISACRYL resulted in the majority of inhibitory activity associating with nonbinding material (fractions 1-30), whereas the bulk of the applied protein (as determined by absorbance at 280 nm,

$$\underline{\hspace{3cm}} )$$

bound to the column resin. These results show that under the conditions of Figure 12, contaminating proteins can be removed from TGI and, therefore, that it is a useful procedure for purification of TGI. In addition, these results show that that at pH 8.0, TGI is a cation since it does not bind the anion exchange resin. Finally, the results of Figure 12 show that TGI as extracted by the modified procedure is similar in ionic character to those polypeptides (TGI-1, TGI-2, CM-I, CM-II, CM-III and CM-IV) extracted by ion exchange resin in the initial procedure since none of these bound to the anion exchange resin.

Large amounts of sample can be reproducibly fractionated by CM-TRISACRYL, thus furnishing more TGI for subsequent purification procedures. In Figure 13, 9.9 mg of tissue extract were applied to a CM-TRISACRYL column (15 ml) under the same chromatographic conditions as shown in Figure 12 for a smaller sample size (2.65 mg protein) on a smaller CM-TRISACRYL column (5 ml). Resolution of TGI activity from the majority of proteinaceous material by a linear gradient of NaCl was essentially the same in both experiments.

Figure 14 shows fractionation of pooled samples (Figure 13, fractions 59-78) from a CM-TRISACRYL column by HPLC on a uBondapac C18 column. Following application of the sample, no significant inhibitory activity was observed by linearly increasing acetonitrile concentrations from 0-25%. However, TGI activity against both A549 (human lung carci noma) and CCL64 (mink lung, 0-0) eluted in a single peak between 28-34% acetonitrile (fractions 21-31) while the majority of material absorbing at 206 nm was eluted at lower (fractions 11-19) and at higher (fractions 37-50) acetonitrile concentrations. The similarities in the activity profile, absorbance at 206nm, and the concentrations of acetonitrile which effectively elute TGI activity between Figure 7 and Figure 14 offer strong evidence that TGI isolated by the modified procedure is similar to or identical to that termed TGI-1 under the First Series of Experiments.

An apparent molecular weight of TGI (termed TGI-1 and CM-III and CM-IV in the initial procedure) was determined by gel filtration chromatography (Sephadex G-50, data not shown) using suitable protein standards of known molecular weights. Thus, in the absence of certain interfering proteins (e.g., hemoglobin) the apparent molecular weight of TGI has been determined to be between 20 kDa and 30 kDa under nondenaturing conditions.

The modified procedure detailed herein describes a powerful and simple procedure for removing inert or interfering compounds from the TGI-containing extracts prepared as described in the initial procedure. Furthermore, the modified procedure improves the efficacy of the various chromatographic steps employed in the isolation of TGI by reducing the amount of chromatographic materials required thus reducing the preparation time of TGI. In addition, and as shown, extraction of TGI from the umbilical cord as described herein allows TGI and other proteins to chromatograph more reproducibly than in the procedure previously described.

TGI isolated according to the modified procedure has been characterized with respect to the chromatographic features on both reverse phase high performance liquid chromatography and CM-TRISACRYL ion exchange chromatography. TGI has been found to behave similarly to or identically with TGI-1 (compare Figures 7 and 14) by rp-HPLC, and thus has similar or identical hydrophobic properties and is shown also to behave similarly to or identically with CM-III and CM-IV (compare Figures 9 and 11) on a cation exchange resin, thus having similar or identical ionic properties. It is therefore concluded that TGI as isolated in the modified procedure and TGI-1 and CM-III and CM-IV are similar or identical compounds having similar or identical ionic and hydrophobic properties and thus are of similar or identical composition. Therefore, the modified procedure described herein provides a more efficacious method of obtaining a purer form of TGI for further analysis and characterization.

**Claims**

1. A composition of matter designated tissue-derived growth inhibitor-1 (TGI-1) which comprises at least one polypeptide having the property of inhibiting the growth of human tumor cells and of an established mink lung cell line (CCL 64) but not the growth of normal human foreskin fibroblasts and having an apparent molecular weight in the range from 5,000-16,000 daltons, the composition:

   a) being derivable from an acidified ethanol extract from human tissue which comprises a plurality of acidic polypeptides, each of which has a molecular weight of less than 20,000 daltons and each of which has the property of inhibiting the growth of human tumor cells and of an established mink lung cell line (CCL 64) while stimulating the growth of normal human foreskin fibroblasts, the inhibitory activity against human tumor cell growth not being destroyed upon increasing the temperature of the acidified, ethanol extract to 100°C for 3 minutes or upon adding acetic acid until the acidified, ethanol extract is up to 1.0 molar in acetic acid and the inhibitory activity being enhanced when the acidified, ethanol extract is prepared at 4°C rather than at 23°C; and

   b) being recoverable as a defined activity on high performance liquid chromatography of an acidified, ethanol extract with a linear gradient of acetonitrile containing 0.05% trifluoroacetic acid at 26-34% acetonitrile and being recoverable as a defined activity on high performance liquid chromatography of the acidified, ethanol extract with a linear gradient of 2-propanol containing 0.05% trifluoroacetic acid at 17-23% 2-propanol.

2. A composition of matter designated tissue-derived growth inhibitor-2 (TGI-2) which comprises at least one polypeptide having the property of inhibiting the growth of human tumor cells and of an established mink lung cell line (CCL 64) but not the growth of normal human foreskin fibroblasts and having an apparent molecular weight in the range from 5,009-16,000 daltons, the composition:

   a) being recoverable as a defined activity on high performance liquid chromatography of an acidified ethanol extract derived from human tissue, the extract comprising a plurality of acidic polypeptides, each of which has an apparent molecular weight less than 20,000 daltons and each of which has the property of inhibiting the growth of human tumor cells and of an established mink lung cell line (CCL 64) while stimulating the growth of normal human foreskin fibroblasts, the inhibitory activity against human tumor cell growth not being destroyed upon increasing the temperature of the acidified, ethanol extract to 100°C for 3 minutes or upon adding acetic acid until the acidified, ethanol extract is up to about 1.0 molar in acetic acid and the inhibitory activity being enhanced when the acidified, ethanol extract is prepared at 4°C rather than at 23°C; and

   b) being recoverable as a defined activity on high, performance liquid chromatography with a linear gradient of acetonitrile containing 0.05% trifluoroacetic acid at 35-39% acetonitrile and being recoverable as a defined activity on high performance liquid chromatography of acidified, ethanol extract with a linear gradient of 2 propanol containing 0.05% trifluoroacetic acid at 23-27% 2-propanol.

3. A pharmaceutical composition comprising an effective amount of the composition of matter of claim 1 and a suitable pharmaceutical carrier.

4. A pharmaceutical composition comprising an effective amount of the composition of flatter of claim 2 and a suitable pharmaceutical carrier.

5. A method for preparing a composition of matter designated tissue-derived growth inhibitor-1 (TGI-1) which comprises first preparing an acidified, ethanol extract and then recovering TGI-1 from the acidified, ethanol extract as a defined activity by high performance liquid chromatography of the acidified, ethanol extract with either i) a linear gradient of acetonitrile containing 0.05% trifluoroacetic acid at 26-34% acetonitrile or ii) a linear gradient of 2-propanol containing 0.05% trifluoroacetic acid at 17-23% 2-propanol, wherein said acidified, ethanol extract is prepared from human tissue, the acidified, ethanol extract comprising a plurality of polypeptides, each of which has a molecular weight less than 20,000 daltons and each of which has the property of inhibiting the growth of human tumor cells and of an established mink lung cell line (CCL 64) while stimulating the growth of normal human foreskin fibroblasts, the method of preparing an acidified, ethanol extract comprising under suitable conditions the following steps:

a. treating the tissue to produce lysed cells and removing the solubilized proteins derived from the cells;

b. recovering the solubilized proteins;

c. separately recovering from the solubilized proteins polypeptides having an apparent molecular weight less than 20,000 daltons;

d. assaying the separately recovered polypeptides to identify those which either inhibit the growth of human tumor cells or inhibit the growth of an established mink lung cell line (CCL 64) or enhance the growth of normal human foreskin fibroblasts; and

e. recovering an acidified, ethanol extract containing the polypeptides so identified.

6. A method for preparing a composition of matter designated tissue-derived growth inhibitor-2 (TGI-2) which comprises first preparing an acidified, ethanol extract and then recovering TGI-2 from the acidified, ethanol extract as a defined activity by high performance liquid chromatography of the acidified, ethanol extract with first i) a linear gradient of acetonitrile containing 0.05% trifluoroacetic acid at 35-37% acetonitrile and then ii) a linear gradient of 2-propanol containing 0.05% trifluoroacetic acid at 23-27% 2-propanol, wherein said acidified, ethanol extract is prepared from human tissue, the acidified, ethanol extract comprising a plurality of polypeptides, each of which has a molecular weight less than 20,000 daltons and each of which has the property of inhibiting the growth of human tumor cells and of an established mink lung cell line (CCL 64) while stimulating the growth of normal human foreskin fibroblasts, the method of preparing an acidified, ethanol extract comprising under suitable conditions the following steps:

a. treating the tissue to produce lysed cells and removing the solubilized proteins derived from the cells;

b. recovering the solubilized proteins;

c. separately recovering from the solubilized proteins polypeptides having an apparent molecular weight less than 20,000 daltons;

d. assaying the separately recovered polypeptides to identify those which either inhibit the growth of human tumor cells or inhibit the growth of an established mink lung cell line (CCL 64) or enhance the growth of normal human foreskin fibroblasts; and

e. recovering an acidified, ethanol extract containing the polypeptides so identified.

7. A method for detecting the presence of a tumor which comprises quantitatively determining the amount of TGI-1 present in a sample from a subject and comparing the amount so determined with the amount present in a sample from a normal subject, the presence of a significantly different amount indicating the presence of a tumor.

8. A method for detecting the presence of a tumor which comprises quantitatively determining the amount of TGI-2 present in a sample from a subject and comparing the amount so determined with the amount present in a sample from a normal subject, the presence of a significantly different amount indicating the presence of a tumor.

9. A method for detecting the presence of a tumor which comprises quantitatively determining the amount of TGI-1 and of transforming growth factor alpha (TGF-alpha) present in a sample from a subject, determining the ratio of the amount of TGI-1 present in the sample to the amount of TGF-alpha, determining the comparable ratio for a sample from a normal subject and comparing the ratio for the normal subject, a significantly different amount indicating the presence of a tumor.

10. A method for detecting the presence of a tumor which comprises quantitatively determining the amount of TGI-2 and of transforming growth factor alpha (TGF-alpha) present in a sample from a subject, determining the ratio of the amount of TGI-2 present in the sample to the amount of TGF-alpha, determining the comparable ratio for a sample from a normal subject and comparing the ratio for the normal subject, a significantly different amount indicating the presence of a tumor.

**Patentansprüche**

1. Material mit der Bezeichnung "aus Gewebe gewonnener Wachstumsinhibitor-1 (TGI-1)", umfassend zumindest ein Polypeptid mit der Eigenschaft, das Wachstum menschlicher Tumorzellen und einer gesicherten Nerzlungenzellinie (CCL 64), jedoch nicht das Wachstum normaler menschlicher Vorhautfibroblasten zu hemmen, und einem scheinbaren Molekulargewicht im Bereich von 5 000 - 16 000 Dalton, das

   a) aus einem angesäuerten Ethanolextrakt menschlichen Gewebes mit einer Mehrzahl von sauren Polypeptiden, von denen jedes ein Molekulargewicht von weniger als 20 000 Dalton aufweist und die Fähigkeit zur Hemmung des Wachstums menschlicher Tumorzellen und einer gesicherten Nerzlungenzellinie (CCL 64) zu hemmen und gleichzeitig das Wachstum normaler menschlicher Vorhautfibroblasten zu stimulieren, besitzt, wobei die Hemmaktivität gegen menschliches Tumorzellenwachstum bei Erhöhen der Temperatur des angesäuerten Ethanolextrakts auf 100°C während 3 min oder bei Zugabe von Essigsäure bis zu 1,0 molaren Sättigung der angesäuerten Ethanolextrakts mit Essigsäure nicht zerstört und darüber hinaus bei Zubereitung des angesäuerten Ethanolextrakts bei 4°C statt bei 23°C verstärkt wird, erhältlich und

   b) als definierte Aktivität bei einer Hochleistungsflüssigchromatographie eines angesäuerten Ethanolextrakts mit einem linearen Gradienten von Acetonitril mit 0,05% Trifluoressigsäure bei 26 - 34% Acetonitril und als definierte Aktivität bei einer Hochleistungsflüssigchromatographie des angesäuerten Ethanolextrakts mit einem linearen Gradienten von 2-Propanol mit 0,05% Trifluoressigsäure bei 17 - 23% 2-Propanol gewinnbar ist.

2. Material mit der Bezeichnung "aus Gewebe gewonnener Wachstumsinhibitor-1 (TGI-1)", umfassend zumindest ein Polypeptid mit der Eigenschaft, das Wachstum menschlicher Tumorzellen und einer gesicherten Nerzlungenzellinie (CCL 64), jedoch nicht das Wachstum normaler menschlicher Vorhautfibroblasten zu hemmen, und einem scheinbaren Molekulargewicht im Bereich von 5 000 - 16 000 Dalton, das

   a) als definierte Aktivität bei einer Hochleistungsflüssigchromatographie eines angesäuerten, aus menschlichem Gewebe gewonnenen Ethanolextrakts, der eine Mehrzahl saurer Polypeptide enthält, von denen jedes ein Molekulargewicht von weniger als 20 000 Dalton aufweist und die Fähigkeit zur Hemmung des Wachstums menschlicher Tumorzellen und einer gesicherten Nerzlungenzellinie (CCL 64) zu hemmen und gleichzeitig das Wachstum normaler menschlicher Vorhautfibroblasten zu stimulieren, besitzt, wobei die Hemmaktivität gegen menschliches Tumorzellenwachstum bei Erhöhen der Temperatur des angesäuerten Ethanolextrakts auf 100°C während 3 min oder bei Zugabe von Essigsäure bis zu 1,0 molaren Sättigung der angesäuerten Ethanolextrakts mit Essigsäure nicht zerstört und darüber hinaus bei Zubereitung des angesäuerten Ethanolextrakts bei 4°C statt bei 23°C verstärkt wird und

   b) als definierte Aktivität bei einer Hochleistungsflüssigchromatographie eines angesäuerten Ethanolextrakts mit einem linearen Gradienten von Acetonitril mit 0,05% Trifluoressigsäure bei 35 - 39% Acetonitril und als definierte Aktivität bei einer Hochleistungsflüssigchromatographie des angesäuerten Ethanolextrakts mit einem linearen Gradienten von 2-Propanol mit 0,05% Trifluoressigsäure bei 23 - 27% 2-Propanol gewinnbar ist.

3. Arzneimittel, enthaltend eine wirksame Menge des Materials gemäß Anspruch 1 und einen geeigneten pharmazeutischen Träger.

4. Arzneimittel, enthaltend eine wirksame Menge des Materials gemäß Anspruch 2 und einen geeigneten pharmazeutischen Träger.

5. Verfahren zur Herstellung eines Materials mit der Bezeichnung "aus Gewebe gewonnener Wachstumsinhibitor-1 (TGI-1)", bei welchem man zunächst einen angesäuerten Ethanolextrakt zubereitet und danach aus dem angesäuerten Ethanolextrakt TGI-I durch Hochleistungsflüssigchromatographie des angesäuerten Ethanolextrakts mittels entweder

   1. eines linearen Gradienten von Acetonitril mit 0,05% Trifluoressigsäure bei 26 - 34% Acetonitril oder

   2. eines linearen Gradienten von 2-Propanol mit 0,05% Trifluoressigsäure bei 17 - 23% 2-Propanol,

   als definierte Aktivität gewinnt, wobei der angesäuerte Ethanolextrakt aus menschlichem Gewebe gewonnen wurde und eine Mehrzahl von Polypeptiden enthält, von denen jedes ein Molekulargewicht von weniger als 20 000 Dalton aufweist und die Fähigkeit zur Hemmung des Wachstums von menschlichen Tumorzellen und einer gesicherten Nerzlungenzellinie (CCL 64) unter Stimulierung des Wachstums normaler menschlicher Vorhautfibroblasten besitzt und wobei man bei der Zubereitung des angesäuerten Ethanolextrakts unter geeigneten Bedingungen folgende Stufen durchführt:

a) Behandeln des Gewebes zur Gewinnung lysierter Zellen und Entfernen der von den Zellen herrührenden löslich gemachten Proteinen;

b) Gewinnen der löslich gemachten Proteine,

c) getrenntes Gewinnen von Polypeptiden eines scheinbaren Molekulargewichts von weniger als 20 000 Dalton aus den löslich gemachten Proteinen;

d) Testen der getrennt gewonnenen Polypeptide zur Identifizierung derjenigen (Polypeptide), die entweder das Wachstum menschlicher Tumorzellen oder das Wachstum einer gesicherten Nerzlungenzellinie (CCL 64) hemmen oder das Wachstum normaler menschlicher Vorhautfibroblasten verstärken; und

e) Gewinnen eines angesäuerten Ethanolextrakts mit den derart identifizierten Polypeptiden.

**6.** Verfahren zur Herstellung eines Materials mit der Bezeichnung "aus Gewebe gewonnener Wachstumsinhibitor-1 (TGI-2)", bei welchem man zunächst einen angesäuerten Ethanolextrakt zubereitet und danach aus dem angesäuerten Ethanolextrakt TGI-I durch Hochleistungsflüssigchromatographie des angesäuerten Ethanolextrakts mittels zunächst

1. eines linearen Gradienten von Acetonitril mit 0,05% Trifluoressigsäure bei 35 - 37% Acetonitril oder

2. eines linearen Gradienten von 2-Propanol mit 0,05% Trifluoressigsäure bei 23 - 27% 2-Propanol,

als definierte Aktivität gewinnt, wobei der angesäuerte Ethanolextrakt aus menschlichem Gewebe gewonnen wurde und eine Mehrzahl von Polypeptiden enthält, von denen jedes ein Molekulargewicht von weniger als 20 000 Dalton aufweist und die Fähigkeit zur Hemmung des Wachstums von menschlichen Tumorzellen und einer gesicherten Nerzlungenzellinie (CCL 64) unter Stimulierung des Wachstums normaler menschlicher Vorhautfibroblasten besitzt und wobei man bei der Zubereitung des angesäuerten Ethanolextrakts unter geeigneten Bedingungen folgende Stufen durchführt:

a) Behandeln des Gewebes zur Gewinnung lysierter Zellen und Entfernen der von den Zellen herrührenden löslich gemachten Proteinen;

b) Gewinnen der löslich gemachten Proteine,

c) getrenntes Gewinnen von Polypeptiden eines scheinbaren Molekulargewichts von weniger als 20 000 Dalton aus den löslich gemachten Proteinen;

d) Testen der getrennt gewonnenen Polypeptide zur Identifizierung derjenigen (Polypeptide), die entweder das Wachstum menschlicher Tumorzellen oder das Wachstum einer gesicherten Nerzlungenzellinie (CCL 64) hemmen oder das Wachstum normaler menschlicher Vorhautfibroblasten verstärken; und

e) Gewinnen eines angesäuerten Ethanolextrakts mit den derart identifizierten Polypeptiden.

**7.** Verfahren zum Nachweis der Anwesenheit eines Tumors durch quantitative Bestimmung der Menge an in einer von einer Testperson entnommenen Probe enthaltenem TGI-1 und Vergleichen der ermittelten Menge mit der in einer von einer normalen Testperson gewonnenen Probe enthaltenen Menge, wobei die Anwesenheit einer signifikant unterschiedlichen Menge ein Anzeichen für das Vorliegen eines Tumors ist.

**8.** Verfahren zum Nachweis der Anwesenheit eines Tumors durch quantitative Bestimmung der Menge an in einer von einer Testperson entnommenen Probe enthaltenem TGI-2 und Vergleichen der ermittelten Menge mit der in einer von einer normalen Testperson gewonnenen Probe enthaltenen Menge, wobei die Anwesenheit einer signifikant unterschiedlichen Menge ein Anzeichen für das Vorliegen eines Tumors ist.

**9.** Verfahren zum Nachweis der Anwesenheit eines Tumors durch quantitative Bestimmung der Menge an TGI-1 und eines transformierenden Wachstumsfaktors Alpha (TGF-alpha) in einer von einer Testperson entnommenen Probe, bestimmung des Verhältnisses der Menge an in der Probe vorhandenem TGI-1 zur der Menge an TGF-alpha, Bestimmen des vergleichbaren Verhältnisses für eine von einer normalen Testperson entnommenen Probe und Vergleichen des Verhältnisses für die normale Testperson, wobei eine signifikant unterschiedliche Menge ein Anzeichen für das Vorhandensein eines Tumors ist.

EP 0 200 090 B1

**10.** Verfahren zum Nachweis der Anwesenheit eines Tumors durch quantitative Bestimmung der Menge an TGI-2 und eines transformierenden Wachstumsfaktors Alpha (TGF-alpha) in einer von einer Testperson entnommenen Probe, bestimmung des Verhältnisses der Menge an in der Probe vorhandenem TGI-1 zur der Menge an TGF-alpha, Bestimmen des vergleichbaren Verhältnisses für eine von einer normalen Testperson entnommenen Probe und Vergleichen des Verhältnisses für die normale Testperson, wobei eine signifikant unterschiedliche Menge ein Anzeichen für das Vorhandensein eines Tumors ist.

**Revendications**

**1.** Composition appelée inhibiteur de croissance d'origine tissulaire 1 (TGI-1) qui comprend au moins un polypeptide ayant la propriété d'inhiber la croissance de cellules tumorales humaines et d'une lignée cellulaire de poumon de vison établie (CCL 64) mais non la croissance de fibroblastes de prépuce humain normaux et ayant un poids moléculaire apparent compris entre 5000 et 16000 daltons, la composition

a) pouvant être obtenus à partir d'un extrait d'éthanol acidifié provenant d'un tissu humain qui comprend plusieurs polypeptides acides, dont chacun a un poids moléculaire inférieur à 20000 daltons et qui ont chacun la propriété d'inhiber la croissance de cellules tumorales humaines et d'une lignée cellulaire de poumon de vison établie (CCL 64) tout en stimulant la croissance de fibroblastes de prépuce humain normaux, l'activité inhibitrice de la croissance cellulaire tumorale humaine n'étant pas détruite lorsqu'on augmente la température de l'extrait d'éthanol acidifié à 100°C pendant 3 minutes ou lorsqu'on ajoute de l'acide acétique jusqu'à ce que l'extrait d'éthanol acidifié soit jusqu'à 1,0 molaire dans l'acide acétique et l'activité inhibitrice étant accrue lorsque l'extrait d'éthanol acidifié est préparé à 4°C plutôt qu'à 23°C; et

b) pouvant être recueillie sous la forme d'une activité définie à la chromatographie en phase liquide haute performance d'un extrait d'éthanol acidifié avec un gradient linéaire d'acétonitrile contenant de l'acide trifluoroacétique à 0,05% à 26-34% d'acétonitrile et pouvant être recueillie sous la forme d'une activité définie à la chromatographie en phase liquide haute performance de l'extrait d'éthanol acidifié avec un gradient linéaire de 2-propanol contenant de l'acide trifluoroacétique à 0,05% à 17-23% de 2-propanol.

**2.** Composition appelée inhibiteur de croissance d'origine tissulaire 2 (TGI-2) qui comprend au moins un polypeptide ayant la propriété d'inhiber la croissance de cellules tumorales humaines et d'une lignée cellulaire de poumon de vison établie (CCL 64) mais non la croissance de fibroblastes do prépuce humain normaux et ayant un poids moléculaire apparent compris entre 5000 et 16000 daltons, la composition:

a) pouvant être recueillie sous la forme d'une activité définie à la chromatographie en phase liquide haute performance d'un extrait d'éthanol acidifié dérivé de tissu humain, l'extrait comprenant plusieurs polypeptides acides, dont chacun a un poids moléculaire apparent inférieur à 20000 daltons etqui ont chacun la propriété d'inhiber la croissance de cellules tumorales humaines et d'une lignée cellulaire de poumon de vison établie (CCL 64) tout en stimulant la croissance de fibroblastes de prépuce humain normaux, l'activité d'inhibition de la croissance des cellules tumorales humaines n'étant pas détruite lorsqu'on augmente la température de l'extrait d'éthanol acidifié à 100°C pendant 3 minutes ou lorsqu'on ajoute de l'acide acétique jusqu'à ce que l'extrait d'éthanol acidifié soit jusqu'à environ 1,0 molaire dans l'acide acétique et l'activité inhibitrice étant accrue lorsque l'extrait d'éthanol acidifié est préparé à 4°C plutôt qu'à 23°C; et

b) pouvant être recueillie sous la forme d'une activité définie à la chromatographie en phase liquide haute performance avec un gradient linéaire d'acétonitrile contenant de l'acide trifluoroacétique à 0,05% à 35-39% d'acétonitrile et pouvant être recueillie sous la forme d'une activité définie à la chromatographie en phase liquide haute performance d'un extrait d'éthanol acidifié avec un gradient linéaire de 2-propanol contenant de l'acide trifluoroacétique à 0,05% à 23-27% de 2-propanol.

**3.** Composition pharmaceutique comprenant une quantité efficace de la composition de la revendication 1 et un support pharmaceutique approprié.

**4.** Composition pharmaceutique comprenant une quantité efficace de la composition de la revendication 2 et un support pharmaceutique approprié

**5.** Procédé de préparation d'une composition appelée inhibiteur de croissance d'origine tissulaire 1 (TGI-

29

1) dans lequel on prépare tout d'abord un extrait d'éthanol acidifié puis on recueille le TGI-1 à partir de l'extrait d'éthanol acidifié sous la forme d'une activité définie par chromatographie en phase liquide haute performance de l'extrait d'éthanol acidifié avec soit i) un gradient linéaire d'acétonitrile contenant de l'acide trifluoroacétique à 0,05% à 26-34% d'acétonitrile, soit ii) un gradient linéaire de 2-propanol contenant de l'acide trifluuoracétique à 0,05% à 17-23% de 2-propanol, où ledit extrait d'éthanol acidifié est préparé à partir de tissu humain, l'extrait d'éthanol acidifié comprenant plusieurs polypeptides dont chacun a un poids moléculaire inférieur à 20000 daltons et dont chacun a la propriété d'inhiber la croissance de cellules tumorales humaines et d'une lignée cellulaire de poumon de vison établie (CCL 64) tout en stimulant la croissance de fibroblastes de prépuce humain normaux, le procédé de préparation d'un extrait d'éthanol acidifié comprenant dans des conditions appropriées les étapes suivantes:

    a. Traitement du tissu pour produire des cellules lysées et enlèvement des protéines solubilisées obtenues à partir des cellules;

    b. Récupération des protéines solubilisées;

    c. Récupération séparée à partir des protéines solubilisées, de polypeptidcs ayant un poids moléculaire apparent inférieur à 20000 daltons;

    d. Dosage des polypeptides recueillis séparément afin d'identifier ceux qui soit inhibent la croissance de cellules tumorales humaines, soit inhibent la croissance d'une lignée cellulaire de poumon de vison établie (CCL 64) , soit augmentent la croissance des fibroblastes de prépuce humain normaux) et

    e. Récupération d'un extrait d'éthanol acidifié contenant les polypeptides ainsi identifiés.

**6.** Procédé de préparation d'une composition appelée inhibiteur de croissance d'origine tissulaire 2 (TGI-2) dans lequel on prépare tout d'abord un extrait d'éthanol acidifié puis on recueille le TGI-2 à partir de l'extrait d'éthanol acidifié sous la forme d'une activité définie par chromatographie en phase liquide haute performance de l'extrait d'éthanol acidifié avec tout d'abord i) un gradient linénire d'acétonitrile contenant de l'acide trifluoroacétique à 0,05% à 35-37% d'acétonitrile, puis ii) un gradient linéaire de 2-propanol contenant de l'acide trifluoroacétique à 0,05% à 23-27% de 2-propanol, où ledit extrait d'éthanol acidifié est préparé à partir de tissu humain, l'extrait d'éthanol acidifié comprenant plusieurs polypeptides dont chacun a un poids moléculaire inférieur à 20000 daltons, et qui chacun ont la propriété d'inhiber la croissance de cellules tumorales humaines et d'une lignée cellulaire de poumon de vison établie (CCL 64) tout en stimulant la croissance de fibroblastes de prépuce humain normaux, le procédé de préparation d'un extrait d'éthanol acidifié comprenant dans des conditions appropriées les étapes suivantes:

    a. Traitement du tissu pour produire des cellules lysées et enlèvement des protéines solubilisées obtenues à partir des cellules;

    b. Récupération des protéines solubilisées;

    c. Récupération séparée à partir des protéines solubilisées, de polypeptides ayant un poids moléculaire apparent inférieur à 20000 daltons;

    d. Dosage des polypeptides recueillis séparément afin d'identifier ceux qui soit inhibent la croissance de cellules tumorales humaines, soit inhibent le croissance d'une lignée cellulaire de poumon de vison établie (CCL 64), soit augmentent la croissance de fibroblastes de prépuce humain normaux; et

    e. Récupération d'un extrait d'éthanol acidifié contenant les polypeptides ainsi identifiés.

**7.** Procédé de détection de la présence d'une tumeur dans lequel on détermine quantitativement la quantité de TGI-1 présente dans un échantillon provenant d'un sujet et on compare la quantité ainsi déterminée avec la quantité présente dans un échantillon provenant d'un sujet normal, la présence d'une quantité significativement différente indiquant la présence d'une tumeur.

**8.** Procédé de détection de la présence d'une tumeur dans lequel on détermine quantitativement la quantité de TGI-2 présente dans un échantillion provenant d'un sujet et on compare la quantité ainsi déterminée avec la quantité présente dans un échantillon provenant d'un sujet normal, la présence d'une quantité significativement différente indiquant la présence d'une tumeur.

**9.** Procédé de détection de la présence d'une tumeur dans lequel on détermine quantitativement la quantité de TGI-1 et de facteur de croissance avec transformation alpha (TGF-alpha) présente dans un échantillon provenant d'un sujet, on détermine la rapport de la quantité de TGF-1 présente dans l'échantillon à la quantité de TGF-alpha, on détermine le rapport comparable pour un échantillon provenant d'un sujet normal et on compare le rapport pour le sujet normal, une quantité significativement différente indiquant la présence d'une tumeur.

10. Procédé de détection de la présence d'une tumeur dans lequel on détermine quantitativement la quantité de TGI-2 et de facteur de croissance avec transformation alpha (TF-alpha) présente dans un échantillon provenant d'un sujet, on détermine le rapport de la quantité de TGI-2 présente dans l'échantillon à la quantité de TGF-alpha, on détermine le rapport comparable pour un échantillon provenant d'un sujet normal et on compare le rapport pour le sujet normal, une quantité significativement différente indiquant la présence d'une tumeur.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14